(19)

**Europäisches Patentamt**
**European Patent Office**
**Office européen des brevets**

(11) **EP 4 660 301 A1**

(12) **EUROPEAN PATENT APPLICATION**
published in accordance with Art. 153(4) EPC

(43) Date of publication:
**10.12.2025 Bulletin 2025/50**

(21) Application number: **24781202.7**

(22) Date of filing: **27.03.2024**

(51) International Patent Classification (IPC):
***C12N 9/48*** (2006.01) ***C12N 15/70*** (2006.01)

(52) Cooperative Patent Classification (CPC):
**C12N 9/48; C12N 15/70**

(86) International application number:
**PCT/KR2024/003809**

(87) International publication number:
**WO 2024/205211 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.03.2023 KR 20230040027**

(71) Applicant: **CJ CheilJedang Corporation**
**Seoul 04560 (KR)**

(72) Inventors:
• **SAGONG, Hye Young**
**Seoul 04560 (KR)**

• **YANG, Tae Joo**
**Seoul 04560 (KR)**
• **CHOI, Eun Jung**
**Seoul 04560 (KR)**
• **SHIN, Sun Mi**
**Seoul 04560 (KR)**

(74) Representative: **Meissner Bolte Partnerschaft mbB**
**Patentanwälte Rechtsanwälte**
**Postfach 86 06 24**
**81633 München (DE)**

Remarks:
The complete document including Reference Table(s) and the Sequence Listing(s) can be downloaded from the EPO website

(54) **MODIFIED POLYPEPTIDE HAVING PROTEASE ACTIVITY**

(57) The present disclosure relates to a variant polypeptide and use thereof.

[FIG. 4]

**Description**

**[Technical Field]**

The present disclosure relates to a variant polypeptide having protease activity and use thereof.

**[Background Art]**

Proteases are enzymes that hydrolyze peptide bonds between amino acids that make up proteins, and are often called peptidases or proteinases. Proteases are used in food processing as tenderizers and meat conditioners, and in medicine as digestive enzymes and thrombolytics. They are also employed as feed additives, research reagents, and in the leather and silk manufacturing industries, as well as being explored as potential therapeutic agents for various applications (US 7,563,872 B2).

**[Disclosure]**

**[Technical Problem]**

Proteases hydrolyze proteins and directly contribute to their removal. Accordingly, they are employed as key enzymes for detergent additives and in biological compound synthesis processes. To secure various industrial applications and economic feasibility, proteases with enhanced catalytic activity or stability are required.

**[Technical Solution]**

It is one object of the present disclosure to provide a variant polypeptide having protease activity.
It is one object of the present disclosure to provide a composition comprising the variant polypeptide.
It is one object of the present disclosure to provide a use of the variant polypeptide or the composition for reacting with peptides.
It is one object of the present disclosure to provide a method for degrading peptide bonds and/or for preparing amino acids, comprising contacting the variant polypeptide, a host cell expressing the variant polypeptide, and/or a composition comprising the variant polypeptide with a substrate.
It is one object of the present disclosure to provide: a polynucleotide encoding the variant polypeptide; a nucleic acid construct comprising the polynucleotide; a vector comprising the polynucleotide or the nucleic acid construct; and/or a host cell comprising the polynucleotide, nucleic acid construct, or vector.
It is one object of the present disclosure to provide a method for preparing the variant polypeptide.

**[Advantageous Effects]**

The variant polypeptide having protease activity of the present disclosure can be usefully employed in various industrial applications.

**[Brief Description of the Drawings]**

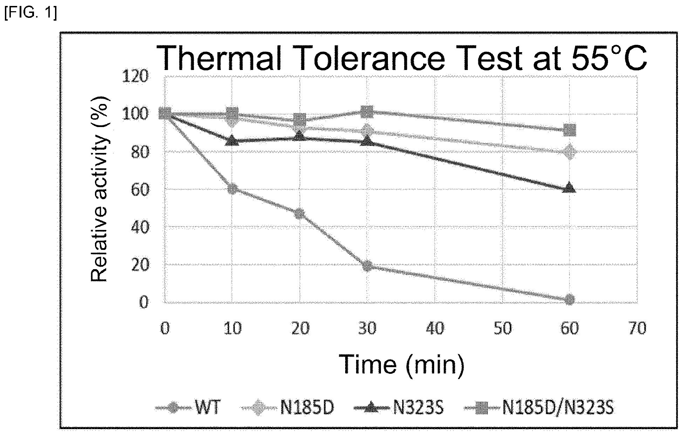

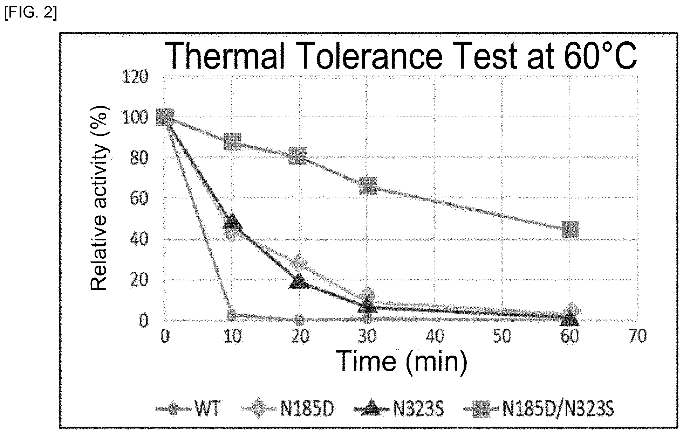

FIGS. 1 and 2 illustrate the results of thermal tolerance tests for WT, N185D, N323S, and a double mutant (N185D/N323S) at 55°C and 60°C, respectively.

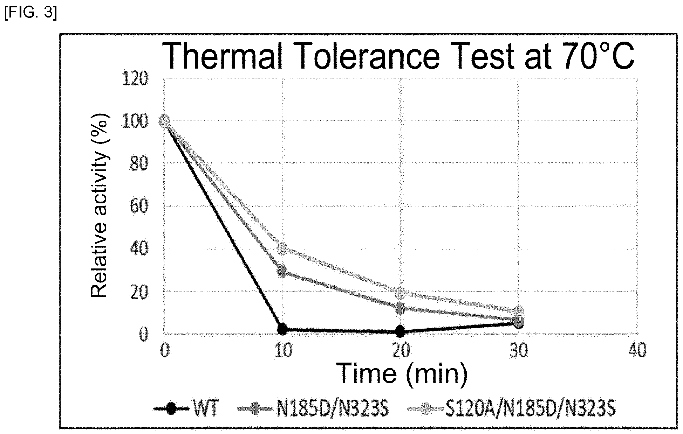

FIG. 3 illustrates the results of thermal tolerance tests for the double mutant (N185D/N323S) and a triple mutant (S120A/N185D/N323S).
FIG. 4 shows the results of thermal stability tests for a JDB4 variant.
FIG. 5 shows the results of thermal stability tests for variants in a PHA deproteinization process.

**[Detailed Description of Preferred Embodiments]**

One aspect of the present disclosure provides a variant polypeptide having protease activity.
In one specific embodiment, i) the variant polypeptide is a polypeptide having a sequence identity of 70% or more and less than 100% with SEQ ID NO: 1; and/or

ii) the variant polypeptide is a polypeptide encoded by a polynucleotide having a sequence identity of 70% or more and less than 100% with the sequence encoding a mature polypeptide of SEQ ID NO: 1; and/or

iii) the variant polypeptide is a polypeptide encoded by a polynucleotide that hybridizes to (a) a mature polypeptide coding sequence of SEQ ID NO: 1, (b) a cDNA thereof, or (c) the full-length complement of (a) or (b) under low stringency conditions, medium stringency conditions, medium-high stringency conditions, high stringency conditions, or very high stringency conditions; and/or

iv) the variant polypeptide is a functional fragment of a polypeptide of i) to iii) having protease activity; and the variant polypeptide comprises any one selected from the following modifications:

deletion, insertion, substitution of an amino acid with another amino acid, and combinations thereof at one or more of positions 120, 153, 185, 293, 323, 349, and 361;
wherein the position number corresponds to the position of the polypeptide of SEQ ID NO: 1.

In one embodiment of any one of the above embodiments, in the variant polypeptide prior to modification: the amino acid at position 120 may be serine (S); the amino acid at position 153 may be asparagine (N); the amino acid at position 185 may be asparagine (N); the amino acid at position 293 may be serine (S); the amino acid at position 323 may be asparagine (N); the amino acid at position 349 may be valine (V); and/or the amino acid at position 361 may be serine (S).

In one embodiment of any one of the above embodiments, the variant polypeptide may comprise modification of amino acids at positions 120, 153, 185, 293, 323, 349, and 361.

In one embodiment of any one of the above embodiments, the variant polypeptide may comprise one or more substitutions selected from the following:

S120A;
N153D;
N185D;
S293P;
N323S;
V349K; and
S361P;
wherein the position number corresponds to the position of the polypeptide of SEQ ID NO: 1.

In one embodiment of any one of the above embodiments, the variant polypeptide may comprise the modification of amino acids at positions selected from i) to x) below:

i) 120;
ii) 185;
iii) 323;
iv)

$$185+323;$$

v)

$$120+185+323;$$

vi)

$$120+153+185+323;$$

vii)

$$120+185+293+323;$$

viii)

$$120+185+323+349;$$

ix)

$$120+185+323+361;$$

and
x)

$$120+153+185+293+323+349+361,$$

wherein the position number corresponds to the position of the polypeptide of SEQ ID NO: 1.

In one embodiment of any one of the above embodiments, the variant polypeptide may comprise one or more substitutions selected from the following:

N185D;
N323S;
N185D+N323S;

$$S120A+N185D+N323S;$$

$$S120A+N153D+N185D+N323S;$$

$$S120A+N185D+S293P+N323S;$$

$$S120A+N185D+N323S+V349K;$$

$$S120A+N185D+N323S+S361P;$$

$$S120A+N153D+N185D+S293P+N323S+V349K+S361P.$$

In one embodiment of any one of the above embodiments, the variant polypeptide may have one or more altered properties compared to a polypeptide consisting of the amino acid sequence of SEQ ID NO: 1, wherein the one or more altered properties are selected from i) to vii) below:

i) increased enzymatic activity;
ii) increased specific activity;
iii) increased pH stability;
iv) increased storage stability;
v) increased acid resistance;
vi) increased thermal tolerance and/or thermal stability; and
vii) altered substrate specificity.

In one embodiment of any one of the above embodiments, the variant polypeptide may have increased thermal tolerance and/or thermal stability compared to a polypeptide consisting of the amino acid sequence of SEQ ID NO: 1.

One aspect of the present disclosure provides a composition comprising the variant polypeptide.

One aspect of the present disclosure provides a use of the variant polypeptide and/or a composition comprising the variant polypeptide for reacting with peptides.

One aspect of the present disclosure provides a method for preparing an amino acid, comprising contacting the variant polypeptide, a host cell expressing the variant polypeptide, and/or a composition comprising the variant polypeptide with a protein or peptide.

One aspect of the present disclosure provides a method for degrading a peptide bond, comprising treating the variant polypeptide, a host cell expressing the variant polypeptide, and/or a composition comprising the variant polypeptide with a substrate.

One aspect of the present disclosure provides a polynucleotide encoding the variant polypeptide.

One aspect of the present disclosure provides a nucleic acid construct comprising the polynucleotide.

One aspect of the present disclosure provides a vector comprising the polynucleotide or the nucleic acid construct.

One aspect of the present disclosure provides a host cell comprising the variant polypeptide, the polynucleotide, the nucleic acid construct, and/or the vector.

One aspect of the present disclosure provides a method for degrading a peptide bond, comprising: culturing the host cell, and recovering the variant polypeptide expressed in the culturing step.

**[Mode for Carrying Out the Invention]**

Hereinafter, the present disclosure will be described in detail. Meanwhile, each description and embodiment disclosed herein can be applied to other descriptions and embodiments, respectively. That is, all combinations of various elements described herein fall within the scope of the present disclosure. Further, the scope of the present disclosure is not limited by the specific description described below.

Furthermore, a person skilled in the art may be able to recognize or identify numerous equivalents to the particular aspects of the present disclosure described herein by using routine experimentation. Moreover, these equivalents are intended to be included in the present disclosure.

As used in the specification and appended claims of the present disclosure, the singular articles ("a," "an," and "the") include plural referents unless the context clearly indicates otherwise. Further, unless the context indicates otherwise, singular terms include their plural forms, and plural terms include their singular forms. As used in the specification and appended claims of the present disclosure, the use of "or" may include the meaning of "and/or", unless indicated otherwise.

As used herein, the term "about" may precede a specific numerical value. As used herein, the term "about" encompasses not only the precise numerical value that is specified after the term but also a range that is approximately or nearly close to that value. Considering the context in which the number is presented, it can be determined whether the specific number mentioned is close to or nearly that number. For example, the term "about" can refer to a range of -10% to +10% of the numerical value. As another example, the term "about" may refer to a range of -5% to +5% of the given numerical value, but is not limited thereto.

As used herein, terms such as "first, second, third, ...", "i), ii), iii), ...", or "(a), (b), (c), (d), ..." are used to distinguish similar elements and do not imply that the elements are executed continuously or in the listed order. For example, when these terms are used in relation to steps of a method, use, or analysis, the steps may be performed without any time intervals, simultaneously, or with intervals of several seconds, minutes, hours, days, or months.

As used herein, the term "consisting essentially of" may mean that, in the case where the features of the object claimed herein are not substantially affected by the presence of an unspecified component, the unspecified component may be present.

As used herein, the term "consisting of" means that the total ratio of specific component(s) is 100%. The components or features that are recited after the term "consisting of" may be essential or mandatory. In some embodiments, any components or non-essential components other than the components or features recited after the term "consisting of" may be excluded.

As used herein, the term "comprising" refers to the presence of features, steps, or components following the term and does not exclude the presence or addition of one or more other features, steps, or components. As used herein, the components or features following the term "comprising" may be essential or mandatory; however, in certain embodiments, other optional or non-essential components or features may also be included.

As used herein, in some embodiments, the term "comprising" may be modified to refer to "consisting essentially of" or "consisting of".

With respect to amino acid sequences in the present disclosure, even if it is disclosed as a polypeptide "comprising" an amino acid sequence described by a specific sequence number, a polypeptide "consisting of" an amino acid sequence described by a specific sequence number, or a polypeptide or protein "having" an amino acid sequence described by a specific sequence number, it is apparent that a protein having an amino acid sequence in which a portion of the sequence is deleted, modified, substituted, conservatively substituted or added can be used in the present disclosure if it has the same or corresponding activity as the polypeptide consisting of the amino acid sequence of the corresponding sequence number. For example, it may be a case where the N-terminus and/or C-terminus of the amino acid sequence is added with a sequence that does not alter the function of the protein, a naturally occurring mutation, or a silent mutation or conservative substitution thereof, but is not limited thereto.

As used herein, the term "protein" or "polypeptide" refers to a polymer or oligomer of consecutive amino acid residues. As used herein, "polypeptide", "protein", and "peptide" may be used interchangeably with "amino acid sequence".

In some cases, an amino acid sequence exhibiting activity may be referred to as an "enzyme". In the present disclosure, unless indicated otherwise, amino acid sequences are described in an N-terminal to C-terminal direction.

As used herein, the term "recombinant" with respect to a cell, nucleic acid, polypeptide, or vector, means that the cell, nucleic acid, polypeptide, or vector has been modified by the introduction of a heterologous nucleic acid or polypeptide, or the alteration of a native nucleic acid or polypeptide, or that the cell is derived from a cell thus modified. Therefore, for example, recombinant cells may express genes that are not found within the native (non-recombinant) form of the cells or may express native genes that are expressed or not expressed, or otherwise abnormally expressed.

As used herein, the term "isolated" refers to a substance that exists in a non-naturally occurring environment or does not exist naturally. It includes at least substantially isolating a substance, for example, a sequence, enzyme, or nucleic acid, that is naturally associated and found in nature from a component having the substance (sequence, enzyme, or nucleic acid).

For example, an isolated sequence, enzyme, or nucleic acid provided herein may be provided in a form that is substantially free of one or more contaminants.

Examples of isolated substances include: i) any non-naturally occurring substance; ii) any substance (e.g., enzyme, variant, nucleic acid, protein, peptide, or cofactor) from which one, or more, or all naturally-occurring components associated in nature have been removed; iii) any substance that has been artificially modified from a substance found in nature; or iv) any substance that has been modified to alter the amount of that substance relative to other components associated in nature (e.g., increase in copy number of a gene encoding a specific substance; modification of a promoter naturally linked to a gene encoding a specific substance into a highly active promoter, *etc.),* but are not limited thereto.

As used herein, the term "wild-type" refers to a naturally-occurring state without artificial modification. When the term "wild-type" is used in reference to a polypeptide, it refers to a naturally occurring polypeptide that does not have artificial mutations (substitutions, insertions, deletions, *etc.*) at one or more amino acid positions. Similarly, when the term "wild-type" is used in reference to a polynucleotide, it refers to a polynucleotide lacking artificial modifications (substitutions, insertions, deletions) of one or more nucleotides. However, polynucleotides encoding wild-type polypeptides are not limited to native polynucleotides and include sequences encoding any wild-type polypeptides.

As used herein, the term "parent sequence" or "backbone" refers to a reference sequence in which modification is introduced to create a modified polypeptide. That is, the parent sequence may be a starting sequence into which modifications such as substitutions, additions, and/or deletions are to be introduced. The parent sequence may be naturally-occurring or wild-type, or a variant in which one or more substitutions, insertions, or deletions have occurred in the natural or wild type, or may be an artificially synthesized sequence. When the parent sequence is an amino acid sequence exhibiting activity, *i.e.,* an amino acid sequence of an enzyme, it may be referred to as a parent enzyme.

As used herein, the term "reference sequence" refers to a sequence used to determine the position of amino acids within any amino acid sequence. By aligning any amino acid sequence with a reference sequence, the position of an amino acid within the sequence that corresponds to a specific position in the reference sequence can be determined.

As used herein, in reference to amino acid or nucleic acid sequences, the term "fragment" refers to a portion of a parent sequence. For example, it may be a polypeptide in the form in which one or more amino acids are removed from the C- or N-terminus of the parent sequence.

As used herein, the "fragment" of an enzyme may refer to a "functional fragment". The term "functional fragment" may also be referred to as an active fragment, and refers to a polypeptide that is a portion of a parent enzyme and has enzymatic activity of the parent enzyme. For example, the functional fragment of an enzyme may include a catalytic site of the enzyme.

A fragment of the enzyme may include a portion of the full length of the parent enzyme. For example, it may include amino acids of about 20%, 30%, 40%, 50%, 60%, 70%, 80%, 90%, 95%, 98%, 99% or more, and less than 100% of the full length of the parent enzyme, but is not limited thereto.

As used herein, "modifying" means changing or altering. It may be altering from a naturally occurring state. For example, an enzyme may be altered in a manner that causes it to differ from a parent sequence or a reference sequence.

In the present disclosure, a modified enzyme may be an enzyme that does not exist in nature by itself, *i.e.,* a non-naturally occurring enzyme.

As used herein, the term "modified" refers, for example, to something that has been altered from its naturally occurring state. The modified enzyme of the present disclosure includes non-naturally occurring enzymes or naturally occurring variants. For example, the modified enzyme of the present disclosure is a modified enzyme that has not been found in nature. For example, the modified enzyme of the present disclosure may be an enzyme that does not occur spontaneously, but is not limited thereto.

As used herein, the term "modification", when used with respect to an amino acid/nucleic acid sequence, may include: substitution of an amino acid/nucleic acid residue of a parent sequence for a different amino acid/nucleic acid residue at one or more positions in the amino acid sequence; deletion of an amino acid/nucleic acid residue (or a series of amino acid/nucleic acid residues) of a parent sequence at one or more positions; insertion of an amino acid/nucleic acid residue (or a series of amino acid/nucleic acid residues) of a parent sequence at one or more positions; truncation of the amino acid sequence at the N-terminus and/or C-terminus or of the nucleic acid sequence at the 5' and/or 3' end; and any combination thereof.

As used herein, a "variant" or "modified polypeptide" of an enzyme refers to a protein having one or more amino acids different from the parent enzyme by conservative substitution and/or modification. The "variant" or "modified polypeptide" may be used interchangeably. The variant or modified polypeptide may be non-naturally occurring, but is not limited thereto.

The variant differs from the sequence of the parent enzyme by one or more modifications, for example, substitutions, deletions, and/or insertions of amino acids.

Such variants may generally be identified by modifying one or more amino acids from the parent enzyme and evaluating the properties of the modified protein. That is, the ability of a variant may be enhanced, unchanged, or reduced relative to the parent enzyme.

Additionally, some variants may include a variant polypeptide in which one or more regions, such as an N-terminal leader sequence or a transmembrane domain, have been removed.

Other variants may include a variant in which a portion has been removed from the N- and/or C-terminus of a mature protein.

The term "variant" or "variant polypeptide" may be used interchangeably with terms including modification, modified protein, mutant, mutein, divergent, and variant, and is not limited as long as the terms are used to indicate mutation.

A variant may also include deletion or addition of amino acids that have minimal influence on the properties and secondary structure of a polypeptide. For example, a polypeptide may be conjugated with a signal (or leader) sequence at the N-terminus of a protein involved in the translocation of proteins co-translationally or post-translationally. Further, the polypeptide may also be conjugated with another sequence or linker to allow for identification, purification, or synthesis of the polypeptide.

As used herein, the term "conservative substitution" refers to the substitution of an amino acid with another amino acid having similar structural and/or chemical properties. Such an amino acid substitution may generally occur based on similarity in polarity, charge, solubility, hydrophobicity, hydrophilicity, and/or amphipathic nature of the residues.

Throughout the entire specification of the present disclosure, the conventional one-letter and three-letter codes for naturally occurring amino acids are used. Additionally, the amino acids mentioned herein are abbreviated according to the IUPAC-IUB nomenclature as follows:

| alanine | Ala, A | arginine | Arg, R |
|---|---|---|---|
| asparagine | Asn, N | aspartic acid | Asp, D |
| cysteine | Cys, C | glutamic acid | Glu, E |
| glutamine | Gln, Q | glycine | Gly, G |
| histidine | His, H | isoleucine | Ile, I |
| leucine | Leu, L | lysine | Lys, K |
| methionine | Met, M | phenylalanine | Phe, F |
| proline | Pro, P | serine | Ser, S |
| threonine | Thr, T | tryptophan | Trp, W |
| tyrosine | Tyr, Y | valine | Val, V |

Meanwhile, any amino acid may be described as Xaa or X.

In addition to naturally occurring amino acids, commonly accepted three-letter codes may also be used for other amino acids such as 2-aminoisobutyric acid (Aib), N-methylglycine (Sar), and $\alpha$-methyl-glutamic acid.

Amino acids can generally be classified based on similarity in polarity, charge, solubility, hydrophobicity, hydrophilicity, and/or amphipathic nature of the residues. Accordingly, amino acid substitution may generally occur based on similarity in polarity, charge, solubility, hydrophobicity, hydrophilicity, and/or amphipathic nature of the residues.

For example, among the amino acids having an electrically charged side chain (electrically charged amino acids), positively charged (basic) amino acids include arginine, lysine, and histidine, and negatively charged (acidic) amino acids include glutamic acid and aspartic acid. Further, among the amino acids having an uncharged side chain (uncharged amino acids), nonpolar amino acids include glycine, alanine, valine, leucine, isoleucine, methionine, phenylalanine, tryptophan, and proline, and polar or hydrophilic amino acids include serine, threonine, cysteine, tyrosine, asparagine, and glutamine. Among the nonpolar amino acids, aromatic amino acids include phenylalanine, tryptophan, and tyrosine.

As used herein, the term "gene" refers to a polynucleotide that encodes a polypeptide and a polynucleotide comprising the upstream and downstream regions of the coding region. In some embodiments, a gene may have a sequence (intron) inserted between each coding region (exon).

As used herein, the term "homology" or "identity" refers to the degree of relevance between two given amino acid sequences or nucleotide sequences, and may be expressed as a percentage. The terms "homology" and "identity" may often be used interchangeably.

The sequence homology or identity of conserved polynucleotides or polypeptides may be determined by a standard alignment algorithm, and default gap penalties established by the program used may be applied. Substantially, homologous or identical sequences may generally hybridize under moderately or highly stringent conditions to the full-length of the sequence or at least about 50%, 60%, 70%, 80%, or 90% or more of the full-length. It is apparent that hybridization also includes hybridization with polynucleotides containing codons that reflect common codon usage or codon degeneracy in the polynucleotides.

Whether any two polynucleotide or polypeptide sequences have homology, similarity, or identity may be determined by, for example, a known computer algorithm such as the "FASTA" program (Pearson et al., (1988) [Proc. Natl. Acad. Sci. USA 85]: 2444) using default parameters. Alternatively, it may be determined by the Needleman-Wunsch algorithm (Needleman and Wunsch, 1970, J. Mol. Biol. 48:443-453) as performed in the Needleman program of the EMBOSS package (EMBOSS: The European Molecular Biology Open Software Suite, Rice et al., 2000, Trends Genet. 16:276-277) (version 5.0.0 or later) (the GCG program package (Devereux, J. et al., Nucleic Acids Research 12:387 (1984)), BLASTP, BLASTN, and FASTA (Atschul, S. F. et al., J MOLEC BIOL 215:403 (1990); Guide to Huge Computers, Martin J. Bishop, ed., Academic Press, San Diego, 1994, and CARILLO et al. (1988) SIAM J Applied Math 48:1073) are included). For example, homology, similarity, or identity may be determined using BLAST from the National Center for Biotechnology Information (NCBI) or ClustalW, but is not limited thereto.

The homology, similarity, or identity of polynucleotides or polypeptides may be determined by comparing sequence information using, for example, a GAP computer program as described in Needleman et al. (1970), J Mol Biol. 48:443, as disclosed in Smith and Waterman, Adv. Appl. Math (1981) 2:482. In summary, the GAP program may be defined as the value obtained by dividing the number of similarly aligned symbols (i.e., nucleotides or amino acids) by the total number of the symbols in the shorter of the two sequences. Default parameters for the GAP program may include: (1) a unitary comparison matrix (containing a value of 1 for identities and 0 for non-identities) and the weighted comparison matrix (or EDNAFULL (EMBOSS version of NCBI NUC4.4) substitution matrix) of Gribskov et al. (1986) Nucl Acids Res. 14:6745, as disclosed in Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or a gap opening penalty of 10 and a gap extension penalty of 0.5); and (3) no penalty for end gaps.

Further, whether any two polynucleotide or polypeptide sequences have homology, similarity, or identity may be identified by comparing the sequences in a Southern hybridization experiment under stringent conditions as defined. Appropriate hybridization conditions defined are within the skill of the art, and may be determined by a method well known to a person skilled in the art (e.g., J. Sambrook et al., Molecular Cloning, A Laboratory Manual, 2nd Edition, Cold Spring Harbor Laboratory press, Cold Spring Harbor, New York, 1989; F. M. Ausubel et al., Current Protocols in Molecular Biology, John Wiley & Sons, Inc., New York). However, the method for identifying homology, similarity, or identity and the appropriate hybridization conditions are not limited thereto.

As used herein, the term "mature polypeptide" refers to a polypeptide in a form without a signal sequence or pro-peptide sequence. A mature protein/polypeptide/peptide may be a functional form of a protein/polypeptide/peptide. A mature polypeptide may be in a final form after translation or post-translational modification. Examples of post-translational modification include N- or C-terminal modification, glycosylation, phosphorylation, removal of a leader sequence, *etc.,* but are not limited thereto.

As used herein, the term "nucleic acid construct" refers to a single- or doublestranded nucleic acid molecule that includes one or more regulatory sequences and is artificially synthesized, engineered to include a specific sequence in a manner that does not exist in nature, or isolated from nature.

As used herein, the term "expression" includes any step involved in the production of a polypeptide, such as transcription, post-transcriptional modification, translation, post-translational modification, and secretion, but is not limited thereto.

As used herein, the term "expression vector" refers to a linear or circular nucleic acid molecule comprising a coding sequence and an operably linked regulatory sequence for expression.

As used herein, the term "operably linked" refers to a configuration in which a regulatory sequence is positioned at an appropriate position to regulate the expression of a coding sequence. Thus, the term "operably linked" includes attaching or linking a regulatory region of a functional domain having a known or desired activity such as a promoter, a stop codon, a signal sequence, or an enhancer region, with a target (gene or polypeptide) so as to regulate the expression, secretion, or function of the target in accordance with the known or desired activity.

As used herein, the term "cDNA" refers to a DNA sequence that can be produced by reverse transcription from a mature, spliced mRNA molecule obtainable from a eukaryotic or prokaryotic cell. A cDNA sequence lacks intron sequences that may be present in the corresponding genomic DNA. The initial primary RNA transcript is a precursor to mRNA that undergoes a series of steps including splicing to become mature spliced mRNA.

As used herein, the term "regulatory sequence" refers to a polynucleotide sequence necessary for the expression of a coding sequence. Each regulatory sequence may be native (derived from the same origin) or foreign (derived from a different gene) to the coding sequence. Examples of the regulatory sequence may include a leader sequence, a polyadenylation sequence, a pro-peptide sequence, a promoter, a signal peptide sequence, an operator sequence, a sequence encoding a ribosome-binding domain, and a sequence regulating the termination of transcription and translation. The minimum unit of the regulatory sequence may include a promoter and a sequence for terminating transcription and translation.

In order to describe the variants provided herein, the following nomenclature is used.

In the present disclosure, referring to a specific position in an amino acid sequence may include referring to an amino acid present or substituted at that position. Referring to an amino acid at a specific position can be described in various ways. For example, "position 003" can be described as "position 3", "amino acid 3", "the third amino acid". Additionally, when the amino acid at position 3 is serine (S), for example, it may be described as "S3" or "Ser3".

Amino acid substitution can be expressed by describing the amino acid before substitution, the position, and the substituted amino acid in sequence. The amino acids may be expressed using the conventional one-letter and three-letter codes. For example, when alanine at position 8 of a specific sequence is substituted with valine, it may be described as "A8V" or "Ala8Val".

Any amino acid at a specific position may be referred to as "X". For example, X6 refers to any amino acid at position 6. Additionally, when the substituted amino acid is expressed as X, it means that it is substituted with an amino acid different from the amino acid before substitution. For example, "V6X" indicates that V at position 6 is substituted with any amino acid other than V.

Different alterations can be expressed by simultaneously describing several types of amino acids using the symbol ",". For example, substitution of an amino acid (D) at position 12 with S or K can be described interchangeably as D12S,K.

Multiple mutations can be described using "/" or "+". For example, descriptions such as "G2A+ M8V" mean that glycine, an amino acid at position 2, and methionine, an amino acid at position 8, are substituted with alanine and valine, respectively. As another example, descriptions such as S120A/N153D mean that serine at position 120 and asparagine at position 153 are substituted with alanine and aspartic acid, respectively.

Amino acid deletion can be expressed by describing the amino acid before deletion, the position, and * in the given order. For example, when alanine, an amino acid corresponding to position 8 of a specific sequence, is deleted, it may be expressed as "A8*" or "(Ala8*)".

Amino acid insertion can be described, for example, as Gly8GlyLys or G8GK, when lysine is inserted between glycine at position 8 and an amino acid at position 9 of a specific sequence. When one or more amino acids are inserted-for example, when lysine and valine are inserted between glycine at position 8 and an amino acid at position 9 of a specific sequence-it can be described as Gly8GlyLysVal or G8GKV. In this case, the positions of the inserted amino acids can be indicated using the amino acid number and alphabet of the amino acid preceding the inserted amino acid. For example, in the case described above, the inserted lysine and valine can be numbered as 8aK and 8bV, respectively.

As used herein, the term "corresponding to" refers to an amino acid residue at the position recited in a protein or peptide, or an amino acid residue that is similar, identical, or homologous to the residue recited in a protein or peptide. Identification of the amino acid at the corresponding position may be determining a specific amino acid in a sequence that refers to a particular sequence. As used herein, the term "corresponding region" refers to a similar or corresponding position in a related protein or a reference protein.

In the present disclosure, SEQ ID NO: 1 can be used as a reference sequence to determine the position of amino acids in any amino acid sequence.

That is, SEQ ID NO: 1 disclosed herein may be used to determine the corresponding amino acid residues in any polypeptide having protease activity. Unless indicated otherwise in the present disclosure, residues of a specific amino acid sequence are numbered relative to SEQ ID NO: 1.

For example, based on the alignment of any amino acid sequence with SEQ ID NO: 1, each amino acid residue in the amino acid sequence can be numbered with reference to the position number of the amino acid residue corresponding to the amino acid residue of SEQ ID NO: 1. For example, a sequence alignment algorithm as described herein can identify the position of an amino acid or a position where modification such as substitution, insertion, or deletion occurs compared to a query sequence (also referred to as the "reference sequence").

An example of the alignment may be determined by the Needleman-Wunsch algorithm (Needleman and Wunsch, 1970, J. Mol. Biol. 48: 443-453), which is performed using the Needleman program of the EMBOSS package (EMBOSS: The European Molecular Biology Open Software Suite, Rice et al., 2000, Trends Genet. 16: 276-277), etc., but the alignment is not limited thereto.

Additionally, multiple sequence alignment can be used to identify corresponding amino acid residues in other proteases. Examples of the multiple sequence alignment known in the art include programs such as MUSCLE (multiple sequence comparison by log-expectation; version 3.5 or later; Edgar, 2004, Nucleic Acids Research 32: 1792-1797), MAFFT (version 6.857 or later; Katoh and Kuma, 2002, Nucleic Acids Research 30: 3059-3066; Katoh et al., 2005, Nucleic Acids Research 33: 511-518; Katoh and Toh, 2007, Bioinformatics 23: 372-374; Katoh et al., 2009, Methods in Molecular Biology 537: 39-64; Katoh and Toh, 2010, Bioinformatics 26: 1899-1900), and EMBOSS EMMA employing ClustalW (1.83 or later; Thompson et al., 1994, Nucleic Acids Research 22: 4673-4680) using their respective default parameters, but the multiple sequence alignment known in the art is not limited thereto.

In addition, when the relationship between enzymes diverged from a mature polypeptide of SEQ ID NO: 1 cannot be identified by traditional sequence-based comparison, other pairwise sequence comparison algorithms may be employed (Lindahl and Elofsson, 2000, J. Mol. Biol. 295: 613-615). Higher sensitivity can be achieved in sequence-based searches by using search programs that utilize probabilistic representations of polypeptide families (profiles) to search databases. For example, the PSI BLAST program can generate profiles through an iterative database search process and detect remote homologs (Atschul et al., 1997, Nucleic Acids Res. 25: 3389-3402). When the family or superfamily of a polypeptide has one or more representatives in protein structure databases, significantly higher sensitivity can be achieved. Programs such as GenTHREADER (Jones, 1999, J. Mol. Biol. 287: 797-815; McGuffin and Jones, 2003, Bioinformatics 19: 874-881) utilize information from a variety of sources, such as PSI BLAST, secondary structure prediction, structural alignment profiles, and solvation potentials, as input to a neural network that predicts the structural fold for a query sequence. Similarly, the method of Gough et al., 2000, J. Mol. Biol. 313: 903-919 may be used to align a sequence of unknown structure with the superfamily models present in the SCOP database. These alignments may be sequentially used to generate homology models for the peptides, and such models can be assessed for accuracy using a variety of tools developed for that purpose.

For proteins of known structure, several tools and resources may be used for retrieving and generating structural alignments. For example, the SCOP superfamilies of proteins have been structurally aligned, and those alignments are accessible and downloadable. Two or more protein structures can be aligned using a variety of algorithms such as the distance alignment matrix (Holm and Sander, 1998, Proteins 33: 88-96) or combinatorial extension (CE) (Shindyalov and Bourne, 1998, Protein Engineering 11: 739-747), and implementation of these algorithms can additionally be utilized to query structure databases with a structure of interest in order to discover possible structural homologs (Holm and Park, 2000, Bioinformatics 16: 566-567).

The above methods are illustrative examples and are not limited thereto.

Hereinafter, the specific embodiments of the present disclosure will be described in more detail.

As used herein, proteases are enzymes that hydrolyze peptide bonds between amino acids that make up proteins or peptides, and are often called peptidases or proteinases.

Proteases are either classified into four types, *i.e.*, serine protease, aspartic protease, cysteine protease, and metalloprotease, according to the main functional group present in the active site or classified into acid protease, neutral protease, and alkaline protease based on the optimal pH of the enzymatic reaction. Proteases have diverse substrates and different cleavage sequences depending on the type, allowing for enzyme-specific substrate engineering, and thereby enabling a wide range of applications.

In one embodiment, the protease of the present disclosure may be an alkaline protease.

As used herein, protease activity can be measured and evaluated using a known method in the art, including the embodiments described in the present disclosure. For example, it can be evaluated by measuring the amount of tyrosine produced by hydrolyzing casein. However, this is merely an example, and the evaluation method is not limited thereto.

As used herein, "parent protease" refers to a protease that is modified to produce the variant or variant polypeptide of the present disclosure. Specifically, the parent protease, parent enzyme, or parent sequence may be a naturally occurring or wild-type polypeptide or a mature polypeptide thereof, and may include a variant or functional fragment thereof. However, it is not limited thereto and may be any polypeptide as long as the polypeptide has protease activity and is capable of serving as a parent of a variant.

The parent protease provided herein may be the polypeptide of SEQ ID NO: 1, but is not limited thereto. Further, the parent protease may be a polypeptide having about 60%, 70%, 75%, 80%, 85%, 90%, 95%, 96%, 97%, 98%, or 99% or more sequence identity with the polypeptide of SEQ ID NO: 1 as long as it exhibits protease activity, and a polypeptide may be included in the scope of the parent protease without limitation if it has the same or equivalent activity as the polypeptide consisting of the amino acid sequence of SEQ ID NO: 1.

The parent protease of the variant provided herein may be derived from an *Alkalihalobacillus* sp. or a *Niallia* sp., and specifically, may be derived from an *Alkalihalobacillus* sp.

For example, it may be derived from *Alkalihalobacillus clausii, Alkalihalobacillus alcalophilus, Alkalihalobacillus xiaoxiensis, Alkalihalobacillus patagoniensis, Alkalihalobacillus lehensis, Alkalihalobacillus pseudalcaliphilus, Alkalihalobacillus okhensis,* or *Alkalihalobacillus shacheensis,* and specifically, may be derived from *Alkalihalobacillus clausii.*

Meanwhile, the above-described microorganisms are merely examples of microorganisms from which the parent protease provided herein can be derived, and microorganisms that are taxonomically homologous thereto, regardless of the name of the microorganism, are included.

The above-described microorganisms may be obtained from known microorganism depositories such as ATCC, DSMZ, CBS, NRRL, KCTC, and KCCM.

As used herein, a sequence "derived from" a specific microorganism is not limited to those naturally produced or producible in the microorganism, but also includes sequences encoded by genes produced and isolated from the microorganism including the genes.

For example, a protease derived from an *Alkalihalobacillus* sp. includes not only enzymes having protease activity that are naturally produced in the *Alkalihalobacillus* sp., but also those produced from an *Alkalihalobacillus* source, and those produced in other host cells through genetic modification known in the art (for example, transformation with a sequence encoding the enzyme).

As used herein, the "variant polypeptide having protease activity" may be a variant of a parent protease.

As used herein, the "variant of a parent protease" or "protease variant" refers to a protein having protease activity with one or more amino acids different from the amino acid sequence of the parent protease.

The "variant polypeptide having protease activity", "variant of a parent protease", and "protease variant" can be used interchangeably.

The variant provided herein may have protease activity and include modifications of one or more amino acids in the sequence of a parent protease. Additionally, the variant may be: i) a polypeptide having a sequence identity of 70% or more and less than 100% with SEQ ID NO: 1; and/or ii) a polypeptide encoded by a polynucleotide having a sequence identity of 70% or more and less than 100% with a sequence encoding a mature polypeptide of SEQ ID NO: 1; and/or iii) a polypeptide encoded by a polynucleotide that hybridizes to (a) a mature polypeptide coding sequence of SEQ ID NO: 1, (b) a cDNA thereof, or (c) the full-length complement of (a) or (b) under low stringency conditions, medium stringency conditions, medium-high stringency conditions, high stringency conditions, or very high stringency conditions; and/or iv) a functional fragment of i), ii), or iii) polypeptide having protease activity.

Specifically, the variant provided herein may have protease activity and include one or more amino acid modifications in the sequence of a parent protease, thereby exhibiting one or more altered functions or properties compared to the parent protease.

In one embodiment, the variant provided herein may have protease activity and include one or more amino acid modifications in the sequence of a parent protease, thereby exhibiting one or more altered functions or properties compared to the parent protease, and may have one or more conserved substitutions.

The variant provided herein is a variant of a parent protease and may be a polypeptide having protease activity.

In one embodiment, the variant provided herein may include modifications at one or more positions corresponding to positions 120, 153, 185, 293, 323, 349, and 361 of SEQ ID NO: 1.

As used herein, the position number corresponds to the position of the polypeptide of SEQ ID NO: 1, and the term "corresponding" is as described above.

In one embodiment, the variant provided herein may include modifications of amino acids corresponding to one or more of S120, N153, N185, S293, N323, V349, and S361 of SEQ ID NO: 1.

In one embodiment, in SEQ ID NO: 1 prior to modification provided herein: the amino acid at position 120 may be serine (S); the amino acid at position 153 may be asparagine (N); the amino acid at position 185 may be asparagine (N); the amino acid at position 293 may be serine (S); the amino acid at position 323 may be asparagine (N); the amino acid at position 349 may be valine (V); and/or the amino acid at position 361 may be serine (S).

In one embodiment, the variant provided herein may include substitution of the amino acid corresponding to position 120 of SEQ ID NO: 1 with G, A, V, L, I, M, F, W, P, T, C, Y, N, Q, D, E, K, R, or H. Specifically, it may include substitution with G, A, V, L, I, M, F, W, or P, and more specifically, substitution with A.

In one embodiment, the variant provided herein may include substitution of the amino acid corresponding to position 153 of SEQ ID NO: 1 with G, A, V, L, I, M, F, W, P, S, T, C, Y, Q, D, E, K, R, or H. Specifically, it may include substitution with D, E, K, R, or H, and more specifically, substitution with D.

In one embodiment, the variant provided herein may include substitution of the amino acid corresponding to position 185 of SEQ ID NO: 1 with G, A, V, L, I, M, F, W, P, S, T, C, Y, Q, D, E, K, R, or H. Specifically, it may include substitution with D, E, K, R, or H, and more specifically, substitution with D.

In one embodiment, the variant provided herein may include substitution of the amino acid corresponding to position 293 of SEQ ID NO: 1 with G, A, V, L, I, M, F, W, P, T, C, Y, N, Q, D, E, K, R, or H. Specifically, it may include substitution with G, A, V, L, I, M, F, W, or P, and more specifically, substitution with P.

In one embodiment, the variant provided herein may include substitution of the amino acid corresponding to position 323 of SEQ ID NO: 1 with G, A, V, L, I, M, F, W, P, S, T, C, Y, Q, D, E, K, R, or H. Specifically, it may include substitution with S, T, C, Y, or Q, and more specifically, substitution with S.

In one embodiment, the variant provided herein may include substitution of the amino acid corresponding to position 349 of SEQ ID NO: 1 with G, A, L, I, M, F, W, P, S, T, C, Y, N, Q, D, E, K, R, or H. Specifically, it may include substitution with D, E, K, R, or H, and more specifically, substitution with K.

In one embodiment, the variant provided herein may include substitution of the amino acid corresponding to position 361 of SEQ ID NO: 1 with G, A, L, I, M, F, W, P, T, C, Y, N, Q, D, E, K, R, or H. Specifically, it may include substitution with G, A, L, I, M, F, W, or P, and more specifically, substitution with P.

In one embodiment, the variant provided herein may include one or more modifications of S120A, N153D, N185D, S293P, N323S, V349K, and S361P of SEQ ID NO: 1.

Specifically, the variant including the substitutions S120A, N153D, N185D, S293P, N323S, V349K, and S361P of SEQ ID NO: 1 may be represented by SEQ ID NO: 2.

In one embodiment, the variant provided herein includes all possible combinations of the above-described modifications.

For example, the variant may include modifications of amino acids at positions selected from the following, as a combination of the above-described modifications:

120;
153;
185;
293;
323;
349;
361;

120 + 153;

120 + 185;

120 + 293;

120 + 323;

120 + 349;

120 + 361;

153 + 185;

153 + 293;

153 + 323;

153 + 349;

153 + 361;

185 + 293;

185 + 323;

185 + 349;

185 + 361;

293 + 323;

293 + 349;

293 + 361;

323 + 349;

323 + 361;

349 + 361;

120 + 153 + 185;

120 + 153 + 293;

120 + 153 + 323;

120 + 153 + 349;

120 + 153 + 361;

120 + 185 + 293;

120 + 185 + 323;

120 + 185 + 349;

120 + 185 + 361;

120 + 293 + 323;

120 + 293 + 349;

120 + 293 + 361;

120 + 323 + 349;

120 + 323 + 361;

120 + 349 + 361;

153 + 185 + 293;

153 + 185 + 323;

153 + 185 + 349;

153 + 185 + 361;

153 + 293 + 323;

153 + 293 + 349;

153 + 293 + 361;

153 + 323 + 349;

153 + 323 + 361;

153 + 349 + 361;

185 + 293 + 323;

185 + 293 + 349;

185 + 293 + 361;

185 + 323 + 349;

185 + 323 + 361;

185 + 349 + 361;

293 + 323 + 349;

293 + 323 + 361;

293 + 349 + 361;

323 + 349 + 361;

120 + 153 + 185 + 293;

120 + 153 + 185 + 323;

120 + 153 + 185 + 349;

120 + 153 + 185 + 361;

120 + 153 + 293 + 323;

120 + 153 + 293 + 349;

120 + 153 + 293 + 361;

120 + 153 + 323 + 349;

120 + 153 + 323 + 361;

120 + 153 + 349 + 361;

120 + 185 + 293 + 323;

120 + 185 + 293 + 349;

120 + 185 + 293 + 361;

120 + 185 + 323 + 349;

120 + 185 + 323 + 361;

120 + 185 + 349 + 361;

120 + 293 + 323 + 349;

120 + 293 + 323 + 361;

120 + 293 + 349 + 361;

120 + 323 + 349 + 361;

153 + 185 + 293 + 323;

153 + 185 + 293 + 349;

153 + 185 + 293 + 361;

153 + 185 + 323 + 349;

153 + 185 + 323 + 361;

153 + 185 + 349 + 361;

153 + 293 + 323 + 349;

153 + 293 + 323 + 361;

153 + 293 + 349 + 361;

153 + 323 + 349 + 361;

185 + 293 + 323 + 349;

185 + 293 + 323 + 361;

185 + 293 + 349 + 361;

185 + 323 + 349 + 361;

293 + 323 + 349 + 361;

120 + 153 + 185 + 293 + 323;

120 + 153 + 185 + 293 + 349;

120 + 153 + 185 + 293 + 361;

120 + 153 + 185 + 323 + 349;

120 + 153 + 185 + 323 + 361;

120 + 153 + 185 + 349 + 361;

120 + 153 + 293 + 323 + 349;

120 + 153 + 293 + 323 + 361;

120 + 153 + 293 + 349 + 361;

120 + 153 + 323 + 349 + 361;

120 + 185 + 293 + 323 + 349;

120 + 185 + 293 + 323 + 361;

120 + 185 + 293 + 349 + 361;

120 + 185 + 323 + 349 + 361;

120 + 293 + 323 + 349 + 361;

153 + 185 + 293 + 323 + 349;

153 + 185 + 293 + 323 + 361;

153 + 185 + 293 + 349 + 361;

$$153 + 185 + 323 + 349 + 361;$$

$$153 + 293 + 323 + 349 + 361;$$

$$185 + 293 + 323 + 349 + 361;$$

$$120 + 153 + 185 + 293 + 323 + 349;$$

$$120 + 153 + 185 + 293 + 323 + 361;$$

$$120 + 153 + 185 + 293 + 349 + 361;$$

$$120 + 153 + 185 + 323 + 349 + 361;$$

$$120 + 153 + 293 + 323 + 349 + 361;$$

$$120 + 185 + 293 + 323 + 349 + 361;$$

$$153 + 185 + 293 + 323 + 349 + 361;$$

$$120 + 153 + 185 + 293 + 323 + 349 + 361.$$

In another example, the variant may include one or more substitutions selected from the following substitutions, but is not limited thereto:

S120A;
N153D;
N185D;
S293P;
N323S;
V349K; and
S361P.

In still another example, the variant may include one or more modifications selected from the following:

S120A;
N153D;
N185D;
S293P;
N323S;
V349K;
S361P;

$$S120A + N153D;$$

$$S120A + N185D;$$

$$S120A + S293P;$$

$$S120A + N323S;$$

S120A + V349K;

S120A + S361P;

N153D + N185D;

N153D + S293P;

N153D + N323S;

N153D + V349K;

N153D + S361P;

N185D + S293P;

N185D + N323S;

N185D + V349K;

N185D + S361P;

S293P + N323S;

S293P + V349K;

S293P + S361P;

N323S + V349K;

N323S + S361P;

V349K + S361P;

S120A + N153D + N185D;

S120A + N153D + S293P;

S120A + N153D + N323S;

S120A + N153D + V349K;

S120A + N153D + S361P;

S120A + N185D + S293P;

S120A + N185D + N323S;

S120A + N185D + V349K;

S120A + N185D + S361P;

S120A + S293P + N323S;

S120A + S293P + V349K;

S120A + S293P + S361P;

S120A + N323S + V349K;

S120A + N323S + S361P;

S120A + V349K + S361P;

N153D + N185D + S293P;

N153D + N185D + N323S;

N153D + N185D + V349K;

N153D + N185D + S361P;

N153D + S293P + N323S;

N153D + S293P + V349K;

N153D + S293P + S361P;

N153D + N323S + V349K;

N153D + N323S + S361P;

N153D + V349K + S361P;

N185D + S293P + N323S;

N185D + S293P + V349K;

N185D + S293P + S361P;

N185D + N323S + V349K;

N185D + N323S + S361P;

N185D + V349K + S361P;

S293P + N323S + V349K;

S293P + N323S + S361P;

S293P + V349K + S361P;

N323S + V349K + S361P;

S120A + N153D + N185D + S293P;

S120A + N153D + N185D + N323S;

S120A + N153D + N185D + V349K;

S120A + N153D + N185D + S361P;

S120A + N153D + S293P + N323S;

S120A + N153D + S293P + V349K;

S120A + N153D + S293P + S361P;

S120A + N153D + N323S + V349K;

S120A + N153D + N323S + S361P;

S120A + N153D + V349K + S361P;

S120A + N185D + S293P + N323S;

S120A + N185D + S293P + V349K;

S120A + N185D + S293P + S361P;

S120A + N185D + N323S + V349K;

S120A + N185D + N323S + S361P;

S120A + N185D + V349K + S361P;

S120A + S293P + N323S + V349K;

S120A + S293P + N323S + S361P;

S120A + S293P + V349K + S361P;

S120A + N323S + V349K + S361P;

N153D + N185D + S293P + N323S;

N153D + N185D + S293P + V349K;

N153D + N185D + S293P + S361P;

N153D + N185D + N323S + V349K;

N153D + N185D + N323S + S361P;

N153D + N185D + V349K + S361P;

N153D + S293P + N323S + V349K;

N153D + S293P + N323S + S361P;

N153D + S293P + V349K + S361P;

N153D + N323S + V349K + S361P;

N185D + S293P + N323S + V349K;

N185D + S293P + N323S + S361P;

N185D + S293P + V349K + S361P;

N185D + N323S + V349K + S361P;

S293P + N323S + V349K + S361P;

S120A + N153D + N185D + S293P + N323S;

S120A + N153D + N185D + S293P + V349K;

S120A + N153D + N185D + S293P + S361P;

S120A + N153D + N185D + N323S + V349K;

S120A + N153D + N185D + N323S + S361P;

S120A + N153D + N185D + V349K + S361P;

S120A + N153D + S293P + N323S + V349K;

S120A + N153D + S293P + N323S + S361P;

S120A + N153D + S293P + V349K + S361P;

S120A + N153D + N323S + V349K + S361P;

S120A + N185D + S293P + N323S + V349K;

S120A + N185D + S293P + N323S + S361P;

S120A + N185D + S293P + V349K + S361P;

S120A + N185D + N323S + V349K + S361P;

S120A + S293P + N323S + V349K + S361P;

N153D + N185D + S293P + N323S + V349K;

N153D + N185D + S293P + N323S + S361P;

N153D + N185D + S293P + V349K + S361P;

N153D + N185D + N323S + V349K + S361P;

N153D + S293P + N323S + V349K + S361P;

N185D + S293P + N323S + V349K + S361P;

S120A + N153D + N185D + S293P + N323S + V349K;

S120A + N153D + N185D + S293P + N323S + S361P;

S120A + N153D + N185D + S293P + V349K + S361P;

S120A + N153D + N185D + N323S + V349K + S361P;

S120A + N153D + S293P + N323S + V349K + S361P;

S120A + N185D + S293P + N323S + V349K + S361P;

N153D + N185D + S293P + N323S + V349K + S361P;

## S120A + N153D + N185D + S293P + N323S + V349K + S361P.

In one embodiment, the variant provided herein may have a sequence identity of about 60% or more, for example, 65% or more, 70% or more, 75% or more, 80% or more, 85% or more, 90% or more, 91% or more, 92% or more, 93% or more, 94% or more, 95% or more, 96% or more, 97% or more, 98% or more, or 99% or more, and less than 100% with the parent protease, a mature polypeptide thereof, or a functional fragment thereof.

In one embodiment, the variant provided herein may have a sequence identity of about 60% or more, for example, 65% or more, 70% or more, 75% or more, 80% or more, 85% or more, 90% or more, 91% or more, 92% or more, 93% or more, 94% or more, 95% or more, 96% or more, 97% or more, 98% or more, or 99% or more, and less than 100% with SEQ ID NO: 1.

In one embodiment, the variant provided herein may be a polypeptide encoded by a polynucleotide having a sequence identity of about 60% or more, for example, 65% or more, 70% or more, 75% or more, 80% or more, 85% or more, 90% or more, 91% or more, 92% or more, 93% or more, 94% or more, 95% or more, 96% or more, 97% or more, 98% or more, or 99% or more, and less than 100% with a sequence encoding a mature polypeptide of SEQ ID NO: 1.

In one embodiment, the variant provided herein may have a sequence identity of about 60% or more, for example, 65% or more, 70% or more, 75% or more, 80% or more, 85% or more, 90% or more, 91% or more, 92% or more, 93% or more, 94% or more, 95% or more, 96% or more, 97% or more, 98% or more, or 99% or more, and less than 100% with a functional fragment of SEQ ID NO: 1.

The variant provided herein may exhibit alterations in one or more selectable or detectable properties or attributes of a polypeptide as compared to other proteases such as wild-type proteases, parent proteases, and other protease variants.

The properties or attributes include oxidative stability, substrate specificity, catalytic activity, thermal stability, alkaline stability, pH activity profile, resistance to proteolytic degradation, Km, $k_{cat}$, $k_{cat}$/Km ratio, protein folding, immune response induction, ability to bind to a ligand, ability to bind to a receptor, ability to be secreted, ability to be displayed on the surface of a cell, ability to oligomerize, ability to send a signal, ability to stimulate cell proliferation, ability to inhibit cell proliferation, ability to induce apoptosis, ability to be modified by phosphorylation or glycosylation, and/or ability to treat diseases, *etc.,* but are not limited thereto.

Specifically, the variant provided herein may have one or more altered activities compared to a parent sequence, selected from the following:

i) increased or decreased enzymatic activity;
ii) increased or decreased specific activity;
iii) increased or decreased pH stability;
iii) increased or decreased storage stability;
v) increased or decreased acid resistance;
vi) increased or decreased thermal tolerance and/or thermal stability; and
vii) altered substrate specificity.

However, the altered activities are not limited thereto.

As used herein, the "enzymatic activity" refers to exhibiting at least one catalytic activity. Specifically, the enzymatic activity may be the conversion efficiency of an enzyme mainly expressed as $k_{cat}$/Km, but is not limited thereto.

$k_{cat}$ refers to the catalytic constant for the rate at which a single enzyme converts substrates into products per unit time when the enzyme is fully saturated with the substrates, and is also called the turnover number. Km refers to the substrate concentration at which the reaction rate is at half the maximum value (Vmax).

Examples of the methods of expressing enzymatic activity include specific activity ($\mu$mol of converted substrate x $mg^{-1}$ x $min^{-1}$) or volumetric activity ($\mu$mol of converted substrate x $mL^{-1}$ x $min^{-1}$).

However, the definition of enzymatic activity is not limited to the description above, and enzymatic activity can be defined and evaluated based on the information disclosed in the following literature: Irwin H. Segel, Enzyme kinetics, John Wiley & Sons, 1979; A. G. Marangoni, Enzyme kinetics, Wiley-Interscience, 2003; A. Fersht, Enzyme structure and mechanisms, John Wiley & Sons, 1981; Structure and Mechanism in Protein Science: A guide to enzyme catalysis and protein folding, Alan Fersht, W.H. Freeman, 1999; Fundamentals of Enzyme Kinetics, Athel Cornish-Bowden, Wiley-Blackwell, 2012; and Voet et al., "Biochemie" [Biochemistry], 1992, VCH-Verlag, Chapter 13, pages 331-332 with respect to enzymatic activity, *etc.*

In one embodiment, the variant provided herein may have increased enzymatic activity by about 100%, about 110%, about 120%, about 130%, about 140%, about 150%, about 160%, about 170%, about 180%, about 190%, or about 200% or more as compared to the parent enzyme.

In another embodiment, the variant provided herein may have decreased enzymatic activity by about 99%, about 95%, about 90%, about 80%, about 70%, about 60%, about 50%, about 40%, or about 20% or less as compared to the parent enzyme.

As used herein, the term "specific activity" refers to the activity of an enzyme per unit weight of protein, and can be expressed as unit/mg. The quantification of protein can be performed using, for example, SDS-PAGE or Bradford assay.

Enzyme stability means that enzymatic activity is maintained during storage or reaction time. In order to measure such changes in stability, the initial enzymatic activity can be measured and compared under defined conditions at time zero (100%) and after a certain period of time (x %), and thus, the level at which the enzymatic activity is lost or enzyme stability can be expressed.

Factors that affect enzymatic activity include, for example, pH, heat, the presence of other substances (e.g., oxidizing agents, chelating agents), *etc.*

As used herein, the term "pH stability" refers to the ability of a protein to function in a specific pH range. In one embodiment, the variant provided herein may have activity at about pH 4.0 to about pH 12.0, but is not limited thereto.

When a protein maintains its function in a specific pH range, it can be defined as having "pH stability", and can also be defined as having "acid resistance", "alkali resistance", *etc.,* depending on the pH range.

As used herein, the term "thermal stability" refers to the ability of a protein to function in a specific temperature range. In one embodiment, the variant provided herein may have activity in the range of about 20°C to about 80°C, but is not limited thereto.

As used herein, the term "thermal tolerance" refers to the ability of a protein to function after exposure to a specific temperature, e.g., high heat or cryogenic temperature. For example, proteins with thermal tolerance may not function at non-optimal temperatures, but may regain their function upon returning to optimal temperature conditions.

Increased stability includes an expanded range of pH, temperature, and/or duration, *etc.,* in which a protein retains its function or maintains high enzymatic activity, as compared to other enzymes such as wild-type enzymes, parent enzymes, and/or other variants.

Decreased stability includes a reduced range of pH, temperature, and/or duration, *etc.,* in which a protein retains its function or maintains low enzymatic activity, as compared to other enzymes such as wild-type enzymes, parent enzymes, and/or other variants.

As used herein, the term "substrate specificity" refers to the ability of an enzyme to identify a substrate and molecules that compete with the substrate. Substrate specificity can be determined by measuring the activity of an enzyme for different substrates. In one embodiment, the change in substrate specificity may be a change in the direction of increasing specificity for a substrate capable of producing a desired product. In another embodiment, the change in substrate specificity may be a change in a direction of decreasing specificity for a substrate capable of producing a desired product.

The altered properties of the variant provided herein may be an activity or an improved activity suitable for application in processes such as detergents, feed, food, research reagents, bio-compound synthesis, and/or removal of protein impurities during purification processes.

The polynucleotide encoding the variant of the present disclosure may include the coding sequence of the above-described variant. The polynucleotide may undergo various modifications in the coding region within the scope that does not change the amino acid sequence of the polypeptide, due to codon degeneracy or in consideration of the codons preferred in an organism in which the polypeptide is to be expressed.

Additionally, the polynucleotide of the present disclosure may include a probe that can be prepared from a known gene sequence, for example, any sequence encoding the variant of the present disclosure by hybridizing with a sequence complementary to all or a portion of the nucleotide sequence under stringent conditions, without limitation.

The term "stringent conditions" refers to conditions that enable specific hybridization between polynucleotides. Such conditions are specifically described in the literature (e.g., J. Sambrook et al., Molecular Cloning, A Laboratory Manual, 2nd Edition, Cold Spring Harbor Laboratory press, Cold Spring Harbor, New York, 1989; F. M. Ausubel et al., Current Protocols in Molecular Biology, John Wiley & Sons, Inc., New York).

For example, the stringent conditions may include conditions under which polynucleotides having a high homology or identity of 40% or more, specifically 90% or more, more specifically 95% or more, 96% or more, 97% or more, or 98% or more, and even more specifically 99% or more, are hybridized with each other and polynucleotides having a homology or identity lower than the above homology or identity are not hybridized with each other, or washing conditions of the conventional Southern hybridization, that is, washing once, specifically, twice or three times at a salt concentration and a temperature corresponding to 60°C, 1×SSC, 0.1% SDS, specifically, 60°C, 0.1×SSC, 0.1% SDS, and more specifically 68°C, 0.1×SSC, 0.1% SDS.

Hybridization requires that two nucleic acids have complementary sequences, although base mismatches are permissible depending on the stringency of the hybridization. The term "complementary" is used to describe a relationship between nucleotide bases that can hybridize with each other. For example, with respect to DNA, adenine is complementary to thymine, and cytosine is complementary to guanine. Therefore, the polynucleotide of the present disclosure may also comprise an isolated nucleic acid fragment complementary to the entire sequence as well as a nucleic acid sequence substantially similar thereto.

Specifically, polynucleotides having homology or identity may be detected using hybridization conditions including hybridization at a Tm value of 55°C, as well as the above-described conditions. Further, the Tm value may be 60°C, 63°C, or 65°C, but is not limited thereto, and may be appropriately adjusted by a person skilled in the art depending on the purpose thereof.

The appropriate stringency for hybridizing polynucleotides depends on the length and degree of complementarity of the polynucleotides, and the relevant parameters are well known in the art (see, Sambrook *et al.,* supra, 9.50-9.51, 11.7-11.8).

For example, "high stringency" may occur at about 5°C to 10°C below the Tm of the probe; "medium stringency" may occur at about 10°C to 20°C below the Tm of the probe; and "low stringency" may occur at about 20°C to 25°C below the Tm, but the stringency is not limited thereto.

For example, the "low stringency condition" may mean, for probes of at least about 100 nucleotides in length, prehybridization and hybridization at 42°C in 5×SSPE, 0.3% SDS, 200 micrograms/ml of sheared and denatured salmon sperm DNA, and 25% formamide, according to the standard Southern blotting procedures for 12 to 24 hours. The carrier substance is finally washed two or three times, each for 15 minutes, in 2×SSC, 0.1% to 0.2% SDS at 50°C.

For example, the "medium stringency condition" may mean, for probes of at least about 100 nucleotides in length, prehybridization and hybridization at 42°C in 5×SSPE, 0.3% SDS, 200 micrograms/ml of sheared and denatured salmon sperm DNA, and 35% formamide, according to the standard Southern blotting procedures for 12 to 24 hours. The carrier substance is finally washed two or three times, each for 15 minutes, in 2×SSC, 0.1% to 0.2% SDS at 55°C. For example, the "medium-high stringency condition" may mean, for probes of at least about 100 nucleotides in length, prehybridization and hybridization at 42°C in 5×SSPE, 0.3% SDS, 200 micrograms/ml of sheared and denatured salmon sperm DNA, and 35% formamide, according to the standard Southern blotting procedures for 12 to 24 hours. The carrier substance is finally washed two or three times, each for 15 minutes, in 2×SSC, 0.1% to 0.2% SDS at 60°C.

For example, the "high stringency condition" may mean, for probes of at least about 100 nucleotides in length, prehybridization and hybridization at 42°C in 5×SSPE, 0.3% SDS, 200 micrograms/ml of sheared and denatured salmon sperm DNA, and 35% formamide, according to the standard Southern blotting procedures for 12 to 24 hours. The carrier substance is finally washed two or three times, each for 15 minutes, in 2×SSC, 0.1% to 0.2% SDS at 65°C.

The nucleic acid construct provided herein may include a polynucleotide encoding the variant provided herein, operably linked to one or more regulatory sequences that direct the expression of the coding sequence in an appropriate host cell under conditions suitable for the regulatory sequences.

The polynucleotide can be engineered in various ways to allow the expression of the variant. Depending on the expression vector, it may be desirable or necessary to engineer the polynucleotide prior to insertion into the vector. Such engineering may be performed using methods known in the art.

The "vector" provided herein refers to a DNA construct containing the nucleotide sequence of a polynucleotide encoding the variant operably linked to a suitable expression regulatory region (or expression regulatory sequence) so as to be able to express the variant of the present disclosure in a suitable host cell. The expression regulatory sequence may include a promoter capable of initiating transcription, any operator sequence for regulating the transcription, a sequence encoding a suitable mRNA ribosome-binding site, and a sequence for regulating termination of transcription and translation. Once transformed into a suitable host cell, the vector may replicate or function independently from the host genome, or may integrate into the genome thereof.

The vector that may be used in the present disclosure is not particularly limited, and any vector known in the art may be used. Examples of commonly used vectors include plasmids, cosmids, viruses, and bacteriophages, either in their natural state or in a recombinant state. For example, as a phage vector or cosmid vector, pWE15, M13, MBL3, MBL4, IXII, ASHII, APII, t10, t11, Charon4A, and Charon21A, *etc.* may be used, and as a plasmid vector, the pBR series, pUC series, pBluescriptII series, pGEM series, pTZ series, pCL series, and pET series, *etc.* may be used. Specifically, pDZ, pACYC177, pACYC184, pCL, pECCG117, pUC19, pBR322, pMW118, and pCC1BAC vectors may be used.

For example, a polynucleotide encoding the variant provided herein may be inserted into the chromosome via a vector for intracellular chromosomal insertion. The insertion of the polynucleotide into the chromosome may be performed by any method known in the art, such as homologous recombination, but is not limited thereto. A selection marker for confirming the chromosomal insertion may be further included. The selection marker is used to screen cells transformed via the vector, that is, to confirm the insertion of the desired nucleic acid molecule. Markers that confer selectable phenotypes, such as drug resistance, nutrient requirements, resistance to cytotoxic agents, or expression of surface polypeptides, may be used. In an environment treated with a selective agent, only the cells that express the selection marker survive or exhibit a distinct phenotype, allowing for the selection of transformed cells.

Any host cell may be included in the host cell of the present disclosure without limitation, as long as it can express the variant of the present disclosure.

The host cell of the present disclosure may include the above-described variant, a polynucleotide encoding the variant, a nucleic acid construct comprising the same and/or a vector comprising the same.

The nucleic acid construct or vector may be integrated into the chromosome as described above, or may be

## EP 4 660 301 A1

maintained as a self-replicating extrachromosomal vector.

The host cell of the present disclosure includes any progeny of a parent cell that is not identical to the parent cell due to mutations occurring during replication.

The host cell may be any cell, for example, a prokaryotic cell or a eukaryotic cell, useful in the recombinant production of a variant.

The prokaryotic host cell may be any Gram-positive or Gram-negative bacterium.

The Gram-positive bacteria include *Bacillus, Clostridium, Enterococcus, Geobacillus, Lactobacillus, Lactococcus, Oceanobacillus, Staphylococcus, Streptococcus,* and *Streptomyces,* but are not limited thereto.

The Gram-negative bacteria include *Campylobacter, Escherichia coli (E. coli), Flavobacterium, Fusobacterium, Helicobacter, Ilyobacter, Neisseria, Pseudomonas, Salmonella, Vibrio (e.g., Vibrio natriegens),* and *Ureaplasma,* but are not limited thereto.

In one embodiment, the bacterial host cell may be a host cell of *Bacillus* sp., specifically including *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* and *Bacillus thuringiensis,* but is not limited thereto.

In one embodiment, the bacterial host cell may be a host cell of *Streptococcus* sp., specifically including *Streptococcus equisimilis, Streptococcus pyogenes, Streptococcus uberis,* and *Streptococcus equi* subsp. *zooepidemicus,* but is not limited thereto.

In one embodiment, the bacterial host cell may be a host cell of *Streptomyces* sp., specifically including *Streptomyces achromogenes, Streptomyces avermitilis, Streptomyces coelicolor, Streptomyces griseus,* and *Streptomyces lividans,* but is not limited thereto.

In one embodiment, the bacterial host cell may be a host cell of *Corynebacterium* sp., and may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens,* but is not limited thereto.

The host cell may be a eukaryotic cell, such as a mammalian, insect, plant, or fungal cell.

The host cell may be a fungal cell. As used herein, "fungi" includes Ascomycota, Basidiomycota, Chytridiomycota, and Zygomycota as well as Oomycota and all mitosporic fungi.

The fungal host cell may be a yeast cell. As used herein, "yeast" includes yeast belonging to ascosporogenous yeasts (Endomycetales), basidiosporogenous yeasts, and Fungi imperfecti (Blastomycetes). However, the classification of yeasts may change, and can be defined as described in Biology and Activities of Yeast (Skinner, Passmore, and Davenport, editors, Soc. App. Bacteriol. Symposium Series No. 9, 1980).

The yeast host cell may be a cell of *Candida, Hansenula, Kluyveromyces, Pichia, Komagataella, Saccharomyces, Schizosaccharomyces,* or *Yarrowia,* such as a cell of *Kluyveromyces lactis, Saccharomyces carlsbergensis, Saccharomyces cerevisiae, Saccharomyces diastaticus, Saccharomyces douglasii, Saccharomyces kluyveri, Saccharomyces norbensis, Saccharomyces oviformis, Komagataella phaffii,* or *Yarrowia lipolytica.*

The fungal host cell may be a filamentous fungal cell. As used herein, "filamentous fungi" include all filamentous forms of the subdivision Eumycota and Oomycota (as defined in Hawksworth *et al.,* 1995, supra). The filamentous fungi are generally characterized by a mycelial wall composed of chitin, cellulose, glucan, chitosan, mannan, and other complex polysaccharides. Vegetative growth occurs through hyphal elongation, and carbon catabolism is strictly aerobic. In contrast, vegetative growth by yeasts, for example, *Saccharomyces cerevisiae,* occurs through the budding of a unicellular thallus, and carbon catabolism may be fermentative.

The filamentous fungal host cell may be a cell of *Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phiebia, Piromyces, Pleurotus, Schizophyllum, Talaromyces, Thermoascus, Thielavia, Tolypocladium, Trametes,* or *Trichoderma.*

For example, the filamentous fungal host cell may be a cell of *Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phiebia radiata, Pleurotus eryngii,*

*Thielavia terrestris, Trametes villosa, Trametes versicolor, Trichoderma harzianum, Trichoderma koningii, Trichoderma longibrachiatum, Trichoderma reesei,* or *Trichoderma viride,* but is not limited thereto.

The composition of the present disclosure may be used to degrade proteins or polypeptides.

The composition of the present disclosure may further comprise other components in addition to the variant provided herein. A person skilled in the art can appropriately select components to be added to the composition of the present disclosure.

In one embodiment, the composition of the present disclosure may further comprise any component suitable for application in converting a protein or peptide into an amino acid.

In one embodiment, the composition of the present disclosure may further comprise any component suitable for application in processes such as detergents, feed, food, research reagents, leather/silk manufacturing industry, pharmaceutical production, biosynthetic compound synthesis, and/or removal of protein impurities during purification. In one embodiment, the bio-compound may be a biodegradable polymer. Examples of the biodegradable polymer include PHA, PLA, PBAT, and PBS.

Examples of substances that may be added include stabilizers, surfactants, builders, chelating agents, dispersants, enzymes, enzyme stabilizers, catalysts, activators, carriers, binders, lubricants, disintegrants, excipients, solubilizers, suspending agents, colorants, flavoring agents, buffers, preservatives, analgesics, solubilizers, isotonic agents, stabilizers, diluents, lubricants, preservatives, *etc.,* but are not limited thereto.

In one embodiment, the composition provided herein may further comprise a naturally occurring substance or a non-naturally occurring substance, in addition to the variant provided herein.

In one embodiment, the composition provided in the present disclosure may further comprise an additional enzyme commonly used in processes such as detergents, feeds, foods, research reagents, bio-compound synthesis, and/or removal of protein impurities during purification, in addition to the variant provided in the present disclosure.

For example, the additional enzyme may further include any one or more enzymes selected from the group consisting of beta-amylase, cellulase (e.g., beta-glucosidase, cellobiohydrolase, and endoglucanase), glucoamylase, hemicellulase *(e.g.,* xylanase), isoamylase, isomerase, lipase, phytase, protease, pullulanase, and/or other enzymes useful in combination with alpha-amylase in a commercial process.

The method for preparing the variant of the present disclosure may include: culturing a host cell; and recovering the variant expressed in the culturing step.

As used herein, the term "culturing" means that the host cell is grown under appropriately controlled environmental conditions. The culturing process of the present disclosure may be performed in a suitable medium known in the art under suitable culturing conditions known in the art. Such a culturing process may be readily adjusted and used by a person skilled in the art according to the selected strain. Specifically, the culturing may be batch culturing, continuous culturing, or fed-batch culturing, but is not limited thereto.

As used herein, the term "medium" refers to a mixed substance containing nutrients required to culture the host cell as a main component, and the medium supplies nutrients, growth factors, etc., including water, which are indispensable for survival and development. Specifically, any medium and culture conditions may be used for culturing the host cell without particular limitation, as long as the medium is used for the common culture of host cells. The host cell may be cultured in a common medium containing suitable carbon sources, nitrogen sources, phosphorus sources, inorganic compounds, amino acids, and/or vitamins, *etc.,* while controlling the temperature, pH, *etc.,* under aerobic conditions.

In the present disclosure, the carbon sources include carbohydrates such as glucose, saccharose, lactose, fructose, sucrose, and maltose; sugar alcohols such as mannitol and sorbitol; organic acids such as pyruvic acid, lactic acid, and citric acid; or amino acids such as glutamic acid, methionine, and lysine. Additionally, natural organic nutrients such as starch hydrolysates, molasses, blackstrap molasses, rice bran, cassava, sugarcane bagasse, and corn steep liquor may be used. Specifically, carbohydrates such as glucose and sterilized pretreated molasses *(i.e.,* molasses converted into reducing sugars) may be used, and other various carbon sources in appropriate amounts may be used without limitation. These carbon sources may be used alone or in combination of two or more, but are not limited thereto.

As the nitrogen sources, inorganic nitrogen sources such as ammonia, ammonium sulfate, ammonium chloride, ammonium acetate, ammonium phosphate, ammonium carbonate, and ammonium nitrate, or organic nitrogen sources such as amino acids (e.g., glutamic acid, methionine, and glutamine), peptone, NZ-amine, meat extracts, yeast extract, malt extract, corn steep liquor, casein hydrolysate, fish or decomposition products thereof, and skim soybean cake or decomposition products thereof may be used. These nitrogen sources may be used alone or in combination of two or more, but are not limited thereto.

As the phosphorus sources, monobasic phosphate, dibasic potassium phosphate, or the corresponding sodium-containing salts may be used. As the inorganic compounds, sodium chloride, calcium chloride, iron chloride, magnesium sulfate, iron sulfate, manganese sulfate, calcium carbonate, *etc.* may be used. In addition, amino acids, vitamins, and/or suitable precursors, *etc.* may be included. These components or precursors may be added to the medium in a batchwise or continuous manner, but are not limited thereto.

During the culturing of the host cell, compounds such as ammonium hydroxide, potassium hydroxide, ammonia, phosphoric acid, and sulfuric acid may be added to the medium in an appropriate manner to adjust the pH of the medium. During the culturing, an antifoaming agent such as fatty acid polyglycol ester may be used to suppress foaming. To maintain an aerobic state of the medium, oxygen or oxygen-containing gas may be injected into the medium. To maintain an anaerobic or microaerobic state of the medium, no gas or nitrogen, hydrogen, or carbon dioxide gas may be injected. However, the culturing conditions are not limited thereto

The temperature during the culturing of the present disclosure may be in the range from 20°C to 40°C, specifically from 25°C to 40°C, but is not limited thereto. The culturing may be carried out until a desired amount of a useful substance is obtained, and may be specifically carried out for 1 to 100 hours, but is not limited thereto.

In one embodiment, the variant expressed in the culturing step may be recovered using a method known in the art. For example, the variant may be recovered from the nutrient medium through a conventional procedure including, but not limited to, collection, centrifugation, filtration, extraction, spray-drying, evaporation, or precipitation.

The recovery method may involve collecting the variant using an appropriate method known in the art based on the culturing method of the host cell of the present disclosure, such as a batch, continuous, or fed-batch culturing method. For example, methods such as centrifugation, filtration, treatment with a protein crystallizing precipitant (salting-out method), extraction, ultrasonic disruption, ultrafiltration, dialysis, various kinds of chromatography such as molecular sieve chromatography (gel filtration), adsorption chromatography, ion exchange chromatography, affinity chromatography, *etc.,* HPLC, and a combination thereof may be used, and the variant can be recovered from the medium or the host cell using suitable methods known in the art.

In another embodiment, the variant expressed by the host cell in the culturing step may not be recovered. In the above embodiment, the host cell expressing the variant may itself be used as a source of the variant.

Peptide bonds can be cleaved using the variant of the present disclosure, a host cell expressing the same, or a composition comprising the variant and/or the host cell. For example, casein may be degraded to form tyrosine.

In addition to the variant of the present disclosure, cofactors, coenzymes, etc. may be added in combination in the hydrolysis step of peptide bonds. The hydrolysis step of a substrate may be performed under optimal pH, temperature conditions, *etc.,* and appropriate conditions can be selected by a person skilled in the art.

Examples

Hereinafter, the present disclosure will be described in more detail by way of Examples and Experimental Examples. However, these Examples and Experimental Examples are given for illustrative purposes only, and the scope of the present disclosure is not intended to be limited by these Examples and Experimental Examples.

Example 1. Screening of Alkaline Protease Variants Derived From *Alkalihalobacillus clausii*

1) Preparation of Alkaline Protease Variants Derived From *Alkalihalobacillus clausii*

(1) Template, Strain, and Medium

Residues predicted to affect the stability of an alkaline protease derived from *Alkalihalobacillus clausii* (SEQ ID NO: 3) were selected, and point mutations were introduced using SDM PCR. A recombinant plasmid pSM704 based on an E. *coli-Bacillus* shuttle vector was used. *E. coli* XL-10 was used as the host cell for gene cloning, and LB broth or agar medium containing ampicillin (final concentration 100 μg/mL) as an antibiotic was used to select transformants. *Bacillus subtilis* LB700 was used for expression and secretion of the improved enzyme protein. For selection of *Bacillus* transformants, LB broth or agar medium containing kanamycin (final concentration 50 μg/mL) as an antibiotic was used.

(2) Production of Variants

To increase the stability of the alkaline protease derived from *Alkalihalobacillus clausii,* residues that affect thermal tolerance were selected through *in silico* design and literature search, and variants were prepared by site-specific substitution of amino acids. To produce recombinant mutants, amino acids were substituted using the site-directed mutagenesis (SDM) method, and a QuikChange Site-Directed Mutagenesis Kit (Agilent Technologies, La Jolla, CA, USA) was used. The gene nucleotide sequences substituted by SDM were confirmed by nucleotide sequence analysis (Macrogen, Seoul).

Information on the primers used is as follows:

[Table 1]

| S120A_F | GTGCCATGGGGGAATTGCCCGTGTGCAAGCC |
|---|---|

(continued)

| | |
|---|---|
| S120A_R | AATTCCCCATGGCACTG |
| N153D_F | CATCCAGACTTAGACATTCGTGGTGGCG |
| N153D_R | CACTCATCCAGACTTA |
| N185D_F | ACGATTGCTGCTTTAGACAATTCGATTGGCGTT |
| N185D_R | TAAAGCAGCAATCGTC |
| S293P_F | AACAACCGCGCCCCATTTTCACAGTATGG |
| S293P_R | GGCGCGGTTGTTGTTT |
| N323S_F | ACGTATGCCAGCTTAAGCGGTACATCGATGGCT |
| N323S_R | TAAGCTGGCATACGTT |
| V349K_F | TCTTGGTCCAATAAACAAATCCGCAAT |
| V349K_R | ATTGGACCAAGATGGG |
| S361P_F | AATACGGCAACGCCATTAGGAAGCACGAA |
| S361P_R | CGTTGCCGTATTCTTT |

(3) Selection of Variants With Improved Thermal Tolerance

Protease activity was measured by detecting tyrosine, an amino acid released from the substrate casein by the enzyme. 0.2 mL of reaction solution contained 0.1 mL of enzyme solution diluted in 50 mM sodium borate buffer (pH 10.5 at room temperature) and 0.1 mL of 1.0% casein (Sigma-Aldrich Co.) as a substrate. After 10 minutes of reaction at 40°C, 0.2 mL of trichloroacetic acid solution (TCA, Sigma-Aldrich Co.) was added to terminate the reaction. After centrifugation (10,000 x g, 10 minutes), 0.75 mL of 0.4 M sodium carbonate anhydrous and 0.15 mL of three-fold diluted Folin-Ciocalteu solution (Sigma-Aldrich Co.) were added to 0.15 mL of the supernatant. The mixture was then subjected to a color development reaction at 40°C for 20 minutes, after which the absorbance was measured at 680 nm using a spectrophotometer. Protease activity was calculated based on a standard curve obtained by quantifying tyrosine (Sigma-Aldrich Co.), and one unit of activity was defined as the amount of enzyme that produces 1 $\mu$g of tyrosine per minute.

(4) Evaluation of Thermal Stability of Variants

Protein activity was evaluated using casein as a substrate for the variants prepared to improve the stability of the alkaline protease derived from *Alkalihalobacillus clausii.* The variants were evaluated to confirm the proteolytic enzyme activity of the enzyme secreted into the *Bacillus* flask culture medium. Proteolytic activity was measured at a wavelength of 680 nm using an analytical method capable of measuring free tyrosine released from casein, and the results are shown in the figures.

JDB variants were prepared using the SDM method, and to evaluate the thermal stability of the selected variants, residual activity was evaluated after heat treatment at 55°C and 60°C for 10, 20, 30, and 60 minutes, respectively. As a result, two single mutants (N185D, N323S) and a combination mutant thereof (N185D/N323S) were identified to exhibit increased thermal stability compared to the wild type.

The thermal stability evaluation was also performed at 70°C, and it was confirmed that the thermal stability of a triple mutant (S120A/N185D/N323S), which includes an additional single mutation, was superior to that of the double mutant (N185D/N323S).

Four residues (N153D, S293P, V349K, S361P) were further identified as affecting the improvement of thermal tolerance. These were added to the triple mutant (S120A/N185D/N323S), and the thermal stability was evaluated at 70°C for 20 minutes. The results are shown in Table 2 below.

[Table 2]

| Triple Mutant | + N153D | + S293P | + V349K | + S361P |
|---|---|---|---|---|
| 100% | 174% | 166% | 138% | 168% |

The experimental results showed that all combination mutants exhibited an improvement in thermal stability by 38% to 74%.

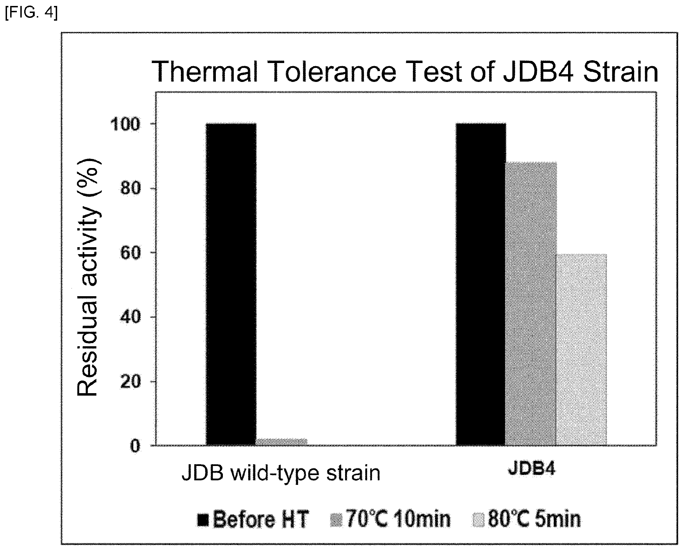

JDB4 (S120A/N153D/N185D/S293P/N323S/V349K/S361P) was produced by further combining the four mutations through the SDM method for combination construction. To evaluate the thermal stability of the selected mutant, residual activity was evaluated before and after heat treatment at 70°C for 10 minutes and 80°C for 5 minutes. As a result, the JDB4 strain was found to exhibit increased stability compared to the wild-type strain (FIG. 4). Specifically, it was found that the mutant JDB4 strain retained 87% activity after heat treatment at 70°C for 10 minutes. While the wild type and the triple mutant strains exhibited no residual activity after heat treatment at 80°C for 5 minutes, the mutant JDB4 strain demonstrated improved thermal stability, retaining 59% activity.

**Example 2. Utilization and Evaluation of Protease Variants in PHA Deproteinization**

1) Utilization of Proteases in a PHA Production Process

The polyhydroxyalkanoate (PHA) production process consists of three steps: 1) microbial fermentation, 2) polymer accumulation in microorganisms, and 3) purification. Removal of impurities during the purification step is a process of removing impurities such as proteins remaining in solid PHA. Proteases are used in the deproteinization step in the PHA production process to selectively degrade proteins attached to PHA granules, thereby reducing the content of impurities within the PHA granules. This step facilitates effective aggregation of PHA microparticles during the acid coagulation process and thus is an important pretreatment step. For an effective deproteinization process, the use of proteases with excellent activity and stability is required.

2) Stability Evaluation Under PHA Deproteinization Conditions

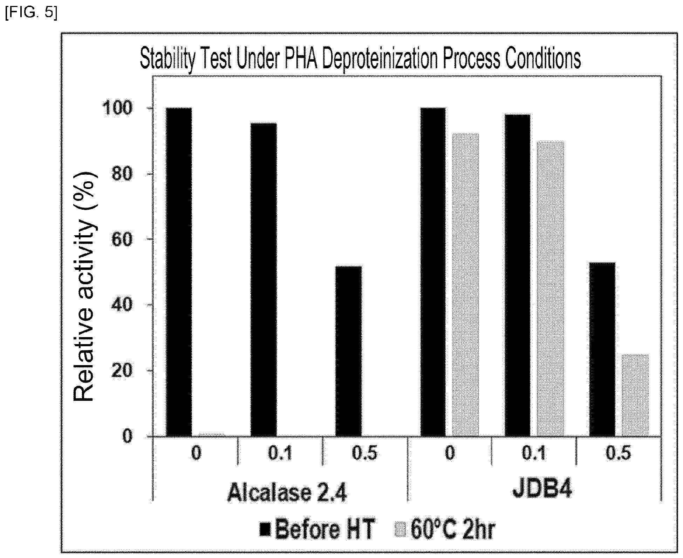

The conditions for the enzymatic deproteinization reaction in the PHA production process are pH 9.5, 60°C for 2 hours, and a small amount of sodium dodecyl sulfate (SDS) is added. For an efficient deproteinization process, it is important to retain enzymatic activity during the reaction. The reaction solution for measuring protease activity consists of 0.1 mL of enzyme solution diluted in 50 mM sodium borate buffer (pH 9.5 at room temperature) and 0.1 mL of 1.0% casein (Sigma-Aldrich Co.) as a substrate. After 10 minutes of reaction at 60°C, 0.2 mL of trichloroacetic acid solution (TCA, Sigma-Aldrich Co.) was added to terminate the reaction. After centrifugation (10,000 X g, 10 minutes), 0.75 mL of 0.4 M sodium carbonate anhydrous and 0.15 mL of three-fold diluted Folin Ciocalteu solution (Sigma-Aldrich Co.) were added to 0.15 mL of the supernatant. The mixture was then subjected to a color development reaction at 40°C for 20 minutes, after which the absorbance was measured at 680 nm using a spectrophotometer. Protease activity was calculated based on a standard curve obtained by quantifying tyrosine (Sigma-Aldrich Co.), and one unit of activity was defined as the amount of enzyme that produces 1 $\mu$g of tyrosine per minute. JDB4 and the commercial product Alcalase 2.4 were subjected to stability evaluation, and the results are shown in FIG. 5.

As a result, the commercial product Alcalase 2.4 showed no residual activity after heat treatment at 60°C for 2 hours. On the other hand, JDB4 demonstrated excellent stability, retaining 92% and 89% activity when 0-0.1% SDS was added, and the residual activity was found to be 24% under conditions where 0.5% SDS was added.

As set forth above, a person skilled in the art will be able to understand that the present disclosure may be embodied in other specific forms without departing from the technical spirit or essential characteristics thereof. Therefore, the embodiments described above should be construed as being exemplified and not limiting the present disclosure. It should be understood that all changes or modifications derived from the definitions and the scopes of the claims and their equivalents fall within the scope of the present disclosure.

**Claims**

1. A variant polypeptide having protease activity, wherein:

   i) the variant polypeptide has a sequence identity of 70% or more and less than 100% with SEQ ID NO: 1; and/or
   ii) the variant polypeptide is a polypeptide encoded by a polynucleotide having a sequence identity of 70% or more and less than 100% with the sequence encoding a mature polypeptide of SEQ ID NO: 1; and/or
   iii) the variant polypeptide is a polypeptide encoded by a polynucleotide that hybridizes to (a) a mature polypeptide coding sequence of SEQ ID NO: 1, (b) a cDNA thereof, or (c) the full-length complement of (a) or (b) under low stringency conditions, medium stringency conditions, medium-high stringency conditions, high stringency conditions, or very high stringency conditions; and/or
   iv) the variant polypeptide is a functional fragment of i), ii), or iii) polypeptide having protease activity; and

   the variant polypeptide comprises any one selected from the following modifications:

deletion, insertion, substitution of an amino acid with another amino acid, and combinations thereof at one or more of positions 120, 153, 185, 293, 323, 349, and 361;
wherein the position number corresponds to the position of the polypeptide of SEQ ID NO: 1.

2. The variant polypeptide according to claim 1, wherein, in the variant polypeptide having protease activity prior to modification: the amino acid at position 120 is serine (S); the amino acid at position 153 is asparagine (N); the amino acid at position 185 is asparagine (N); the amino acid at position 293 is serine (S); the amino acid at position 323 is asparagine (N); the amino acid at position 349 is valine (V); and/or the amino acid at position 361 is serine (S).

3. The variant polypeptide according to claim 1, wherein the variant polypeptide comprises modifications of amino acids at positions selected from i) to x) below:

i) 120;
ii) 185;
iii) 323;
iv)

185+323;

v)

120+185+323;

vi)

120+153+185+323;

vii)

120+185+293+323;

viii)

120+185+323+349;

ix)

120+185+323+361;

and
x)

120+153+185+293+323+349+361,

wherein the position number corresponds to the position of the polypeptide of SEQ **ID** NO: 1.

4. The variant polypeptide according to claim 1, wherein the variant polypeptide comprises one or more substitutions selected from i) to vii) below:

i) substitution of the amino acid at position 120 with alanine;
ii) substitution of the amino acid at position 153 with aspartic acid;
iii) substitution of the amino acid at position 185 with aspartic acid;
iv) substitution of the amino acid at position 293 with proline;
v) substitution of the amino acid at position 323 with serine;
vi) substitution of the amino acid at position 349 with lysine; and
vii) substitution of the amino acid at position 361 with proline,

wherein the position number corresponds to the position of the polypeptide of SEQ **ID** NO: 1.

5. The variant polypeptide according to claim 1, wherein the variant polypeptide comprises one or more substitutions selected from the following:

S120A;
N153D;
N185D;
S293P;
N323S;
V349K; and
S361P;
wherein the position number corresponds to the position of the polypeptide of SEQ ID NO: 1.

6. The variant polypeptide according to claim 1, wherein the variant polypeptide comprises the modification S120A/N153D/N185D/S293P/N323S/V349K/S361P.

7. The variant polypeptide according to claim 1, wherein the variant polypeptide has one or more altered properties compared to the polypeptide consisting of the amino acid sequence of SEQ ID NO: 1, wherein the one or more altered properties are selected from i) to vii) below:

i) increased enzymatic activity;
ii) increased specific activity;
iii) increased pH stability;
iv) increased storage stability;
v) Increased acid resistance;
vi) increased thermal tolerance and/or thermal stability; and
vii) altered substrate specificity.

8. A composition comprising the variant polypeptide of any one of claims 1 to 7.

9. Use of a variant polypeptide according to any one of claims 1 to 7 or a composition comprising the variant polypeptide for degrading peptide bonds.

10. A method for degrading a peptide bond, comprising treating a substrate with the variant polypeptide of any one of claims 1 to 7, a host cell expressing the variant polypeptide, and/or a composition comprising the variant polypeptide.

11. A polynucleotide encoding the variant polypeptide of any one of claims 1 to 7.

12. A host cell comprising the variant polypeptide of any one of claims 1 to 7 and/or the polynucleotide of claim 11.

13. A method for preparing a variant polypeptide having protease activity, comprising:

culturing the host cell of claim 12; and
recovering the variant polypeptide having protease activity of any one of claims 1 to 7 expressed in the culturing step.

[FIG. 1]

[FIG. 2]

Thermal Tolerance Test at 60°C

[FIG. 3]

[FIG. 4]

**Thermal Tolerance Test of JDB4 Strain**

Residual activity (%) vs. JDB wild-type strain and JDB4

Legend: ■ Before HT  ▨ 70°C 10min  ▨ 80°C 5min

[FIG. 5]

## INTERNATIONAL SEARCH REPORT

| International application No. |
| --- |
| **PCT/KR2024/003809** |

### A. CLASSIFICATION OF SUBJECT MATTER

**C12N 9/48**(2006.01)i; **C12N 15/70**(2006.01)i

According to International Patent Classification (IPC) or to both national classification and IPC

### B. FIELDS SEARCHED

Minimum documentation searched (classification system followed by classification symbols)

C12N 9/48(2006.01); C11D 3/386(2006.01); C12N 15/09(2006.01); C12N 15/63(2006.01); C12N 9/50(2006.01); C12N 9/54(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 프로테아제(protease), 알칼리할로바실러스 속(alkalihalobacillus sp.), 폴리펩티드 (polypeptide), 결실(deletion), 삽입(insertion), 치환(substitution), 변형(modification), S120A, N153D, N185D, S293P, N323S, V349K, S361P

### C. DOCUMENTS CONSIDERED TO BE RELEVANT

| Category* | Citation of document, with indication, where appropriate, of the relevant passages | Relevant to claim No. |
| --- | --- | --- |
| A | US 5453372 A (VETTER, R. et al.) 26 September 1995 (1995-09-26) See abstract; claims 1-2; column 3, line 64 - column 4, line 57; SEQ ID NO: 2; and figure 1. | 1-11 |
| A | NCBI Reference Sequence : WP_094423791.1 (10 July 2019). See entire document. | 1-11 |
| A | US 6271012 B1 (VAN EEKELEN, C. A. G. et al.) 07 August 2001 (2001-08-07) See entire document. | 1-11 |
| A | WO 2017-213168 A1 (KAO CORPORATION) 14 December 2017 (2017-12-14) See entire document. | 1-11 |
| A | US 2012-0058928 A1 (TOHATA, M. et al.) 08 March 2012 (2012-03-08) See entire document. | 1-11 |

☐ Further documents are listed in the continuation of Box C.  ☑ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **25 June 2024** | **26 June 2024** |

| Name and mailing address of the ISA/KR | Authorized officer |
| --- | --- |
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

International application No.

**PCT/KR2024/003809**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a.   ☑   forming part of the international application as filed.

    b.   ☐   furnished subsequent to the international filing date for the purposes of international search (Rule 13*ter*.1(a)),

         ☐   accompanied by a statement to the effect that the sequence listing does not go beyond the disclosure in the international application as filed.

2.   ☐   With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.

**PCT/KR2024/003809**

| Box No. II | Observations where certain claims were found unsearchable (Continuation of item 2 of first sheet) |

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☑ Claims Nos.: **12-13**
because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

International application No.

**PCT/KR2024/003809**

| Patent document cited in search report | | | Publication date (day/month/year) | Patent family member(s) | | | Publication date (day/month/year) |
|---|---|---|---|---|---|---|---|
| US | 5453372 | A | 26 September 1995 | EP | 0525610 | A2 | 03 February 1993 |
| | | | | EP | 0995801 | A1 | 26 April 2000 |
| US | 6271012 | B1 | 07 August 2001 | CN | 1036602 | A | 25 October 1989 |
| | | | | EP | 0328229 | A1 | 16 August 1989 |
| | | | | EP | 0328229 | B1 | 19 January 1994 |
| | | | | EP | 0328229 | B2 | 30 October 1996 |
| | | | | EP | 0571049 | A1 | 24 November 1993 |
| | | | | EP | 0571049 | B1 | 24 September 2003 |
| | | | | EP | 0571049 | B2 | 03 September 2008 |
| | | | | EP | 0717778 | A1 | 26 June 1996 |
| | | | | EP | 0717778 | B1 | 17 October 2007 |
| | | | | EP | 1160327 | A2 | 05 December 2001 |
| | | | | EP | 1160327 | A3 | 06 November 2002 |
| | | | | JP | 02-503986 | A | 22 November 1990 |
| | | | | JP | 07-508887 | A | 05 October 1995 |
| | | | | JP | 2849141 | B2 | 20 January 1999 |
| | | | | JP | 4028592 | B2 | 26 December 2007 |
| | | | | KR | 10-1995-0702633 | A | 29 July 1995 |
| | | | | KR | 10-1997-0005249 | B1 | 14 April 1997 |
| | | | | US | 5324653 | A | 28 June 1994 |
| | | | | US | 5336611 | A | 09 August 1994 |
| | | | | US | 6287841 | B1 | 11 September 2001 |
| | | | | WO | 89-07642 | A1 | 24 August 1989 |
| | | | | WO | 94-02618 | A1 | 03 February 1994 |
| WO | 2017-213168 | A1 | 14 December 2017 | CN | 109312323 | A | 05 February 2019 |
| | | | | CN | 109312323 | B | 08 March 2022 |
| | | | | EP | 3470517 | A1 | 17 April 2019 |
| | | | | EP | 3470517 | B1 | 10 January 2024 |
| | | | | JP | 2017-221188 | A | 21 December 2017 |
| | | | | JP | 6289711 | B2 | 07 March 2018 |
| | | | | US | 10717949 | B2 | 21 July 2020 |
| | | | | US | 2019-0161708 | A1 | 30 May 2019 |
| US | 2012-0058928 | A1 | 08 March 2012 | CN | 102421893 | A | 18 April 2012 |
| | | | | CN | 102421893 | B | 29 October 2014 |
| | | | | EP | 2424981 | A2 | 07 March 2012 |
| | | | | EP | 3061817 | A1 | 31 August 2016 |
| | | | | EP | 3061817 | B1 | 12 December 2018 |
| | | | | JP | 2010-273672 | A | 09 December 2010 |
| | | | | JP | 2010-273673 | A | 09 December 2010 |
| | | | | US | 8778650 | B2 | 15 July 2014 |
| | | | | WO | 2010-126156 | A2 | 04 November 2010 |
| | | | | WO | 2010-126156 | A3 | 06 January 2011 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 7563872 B2

### Non-patent literature cited in the description

- **PEARSON et al.** *Proc. Natl. Acad. Sci. USA*, 1988, vol. 85, 2444
- **NEEDLEMAN ; WUNSCH**. *J. Mol. Biol.*, 1970, vol. 48, 443-453
- **RICE et al.** *Trends Genet*, 2000, vol. 16, 276-277
- **DEVEREUX, J et al.** *Nucleic Acids Research*, 1984, vol. 12, 387
- **ATSCHUL, S. F. et al.** *J MOLEC BIOL*, 1990, vol. 215, 403
- Guide to Huge Computers. Academic Press, 1994
- **CARILLO et al.** *SIAM J Applied Math*, 1988, vol. 48, 1073
- **NEEDLEMAN et al.** *J Mol Biol*, 1970, vol. 48, 443
- **SMITH ; WATERMAN**. *Adv. Appl. Math*, 1981, vol. 2, 482
- **GRIBSKOV et al.** *Nucl Acids Res*, 1986, vol. 14, 6745
- Atlas Of Protein Sequence And Structure. National Biomedical Research Foundation, 1979, 353-358
- **J. SAMBROOK et al.** Molecular Cloning, A Laboratory Manual. Cold Spring Harbor Laboratory press, 1989
- **F. M. AUSUBEL et al.** Current Protocols in Molecular Biology. John Wiley & Sons, Inc.
- **RICE et al.** EMBOSS: The European Molecular Biology Open Software Suite. *Trends Genet*, 2000, vol. 16, 276-277
- **EDGAR**. *Nucleic Acids Research*, 2004, vol. 32, 1792-1797
- **KATOH ; KUMA**. *Nucleic Acids Research*, 2002, vol. 30, 3059-3066
- **KATOH et al.** *Nucleic Acids Research*, 2005, vol. 33, 511-518
- **KATOH ; TOH**. *Bioinformatics*, 2007, vol. 23, 372-374
- **KATOH et al.** *Methods in Molecular Biology*, 2009, vol. 537, 39-64
- **KATOH ; TOH**. *Bioinformatics*, 2010, vol. 26, 1899-1900
- **THOMPSON et al.** *Nucleic Acids Research*, 1994, vol. 22, 4673-4680
- **LINDAHL ; ELOFSSON**. *J. Mol. Biol.*, 2000, vol. 295, 613-615
- **ATSCHUL et al.** *Nucleic Acids Res.*, 1997, vol. 25, 3389-3402
- **JONES**. *J. Mol. Biol.*, 1999, vol. 287, 797-815
- **MCGUFFIN ; JONES**. *Bioinformatics*, 2003, vol. 19, 874-881
- **GOUGH et al.** *J. Mol. Biol.*, 2000, vol. 313, 903-919
- **HOLM ; SANDER**. *Proteins*, 1998, vol. 33, 88-96
- **SHINDYALOV ; BOURNE**. *Protein Engineering*, 1998, vol. 11, 739-747
- **HOLM ; PARK**. *Bioinformatics*, 2000, vol. 16, 566-567
- **IRWIN H. SEGEL**. Enzyme kinetics. John Wiley & Sons, 1979
- **A. G. MARANGONI**. Enzyme kinetics. Wiley-Interscience, 2003
- **A. FERSHT**. Enzyme structure and mechanisms. John Wiley & Sons, 1981
- **ALAN FERSHT ; W.H. FREEMAN**. *Structure and Mechanism in Protein Science: A guide to enzyme catalysis and protein folding*, 1999
- **ATHEL CORNISH-BOWDEN**. Fundamentals of Enzyme Kinetics. Wiley-Blackwell, 2012
- **VOET et al.** Biochemie" [Biochemistry. VCH-Verlag, 1992, 331-332
- Soc. App. Bacteriol. Symposium Series. 1980, vol. 9